(19) Europäisches Patentamt
European Patent Office
Office européen des brevets

(11) **EP 3 256 036 B1**

(12) **EUROPÄISCHE PATENTSCHRIFT**

(45) Veröffentlichungstag und Bekanntmachung des Hinweises auf die Patenterteilung:
**29.07.2020 Patentblatt 2020/31**

(21) Anmeldenummer: **16703429.7**

(22) Anmeldetag: **26.01.2016**

(51) Int Cl.:
*A61B 3/00* (2006.01)    *G02C 13/00* (2006.01)
*A61B 3/15* (2006.01)    *A61B 3/14* (2006.01)

(86) Internationale Anmeldenummer:
**PCT/EP2016/000133**

(87) Internationale Veröffentlichungsnummer:
**WO 2016/128112 (18.08.2016 Gazette 2016/33)**

(54) **VORRICHTUNG UND VERFAHREN ZUR ABSTANDSBESTIMMUNG UND/ODER ZENTRIERUNG UNTER VERWENDUNG VON HORNHAUTREFLEXIONEN**

DEVICE AND METHOD FOR DISTANCE DETERMINATION AND/OR CENTERING USING CORNEAL REFLECTIONS

DISPOSITIF ET PROCÉDÉ DE DÉTERMINATION DE DISTANCE ET/OU DE CENTRAGE À L'AIDE DES RÉFLEXIONS CORNÉENNES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.02.2015 DE 102015001874**

(43) Veröffentlichungstag der Anmeldung:
**20.12.2017 Patentblatt 2017/51**

(73) Patentinhaber: **Rodenstock GmbH**
**80687 München (DE)**

(72) Erfinder:
• **Dr. SEITZ, Peter**
  **71272 Renningen (DE)**
• **TIEMANN, Markus**
  **81539 München (DE)**
• **ESSER, Gregor**
  **80686 München (DE)**
• **SEIDEMANN, Anne**
  **81375 München (DE)**
• **MÜLLER, Werner**
  **75443 Ötisheim (DE)**

(74) Vertreter: **Müller-Boré & Partner**
**Patentanwälte PartG mbB**
**Friedenheimer Brücke 21**
**80639 München (DE)**

(56) Entgegenhaltungen:
EP-A1- 2 653 094      WO-A1-2008/009355
WO-A1-2014/103646    DE-A1-102005 038 218

**Beschreibung**

Die vorliegende Erfindung betrifft eine Vorrichtung umfassend eine Bildaufnahmeeinrichtung und eine Messeinrichtung zum Bestimmen des Abstands eines Auges oder beider Augen eines Benutzers von der Bildaufnahmeeinrichtung (wie einer Kamera). Die Vorrichtung kann eine Vorrichtung zum Bestimmen von optischen Parametern eines Benutzers, eine Vorrichtung zum Erstellen von Stereobildern und/oder zum Bestimmen von Ortsinformationen im dreidimensionalen Raum, eine Vorrichtung zum Anzeigen von Inhalten in Abhängigkeit von der Lage eines Benutzers, etc. sein. Ferner betrifft die vorliegende Erfindung ein Verfahren zum Bestimmen des Abstands eines Auges oder beider Augen eines Benutzers von einer Bildaufnahmeeinrichtung und ein Computerprogrammprodukt zum Durchführen des Verfahrens.

Aus dem Stand der Technik sind beispielsweise Videozentriervorrichtungen bekannt, die einzelne oder auch alle Parameter der Augen eines Benutzers und/oder der Gebrauchsstellung einer Brille oder Brillenfassung vor den Augen eines Benutzers durch Aufnahme eines Bildes des Benutzers und eventuell unter Zuhilfenahme von weiteren Hilfsmitteln bestimmen können. Die Parameter der Augen und/oder der Gebrauchsstellung umfassen z.B. die Pupillendistanz des Benutzers, den Fassungsscheibenwinkel einer vom Benutzer getragenen Brille oder Brillenfassung, die Brillenglasvorneigung, die Form der Brillenfassung, den Hornhautscheitelabstand des Systems von Brille und Auge, die Einschleifhöhe der Brillengläser, etc.

Die Druckschrift DE 10 2005 003 699 A1 offenbart ein Stereokamerasystem mit zwei Kameras, die aus unterschiedlichen Aufnahmerichtungen jeweils ein Bild von einem Benutzer mit Brille aufnehmen, aus denen dreidimensionale Benutzerdaten errechnet werden. Es sind ebenfalls Videozentriervorrichtungen mit einer Kamera bekannt, welche zeitlich versetzte Aufnahmen der Augenpartie des zu vermessenden Benutzers aus verschiedenen Blickwinkeln machen. Die Aufnahmen werden nachträglich unter Zuhilfenahme eines Referenzobjekts im Bild (z.B. eines Zentrierbügels) zueinander orientiert, so dass ebenfalls ein Stereokamerasystem gebildet wird. Andere Lösungen verwenden nur die mit einer Kamera aufgenommene Projektion im Gesicht eines Benutzers einer Fassung, auf der ein Zentrierbügel angebracht ist. Durch eine Annahme von Werten des Hornhautscheitelabstands, einer Vorneigungsdifferenz zwischen Zentrierbügel und Fassung und eines Fassungsscheibenwinkels können einige Parameter näherungsweise bestimmt werden.

Die mittels einer Videozentriervorrichtung bestimmten Parameter der Augen und/oder der Gebrauchsstellung einer Brille oder Brillenfassung vor den Augen eines Benutzers können einerseits bei dem Einschleifen und Einsetzen von Brillengläsern in eine Brillenfassung berücksichtigt werden, andererseits können damit Optimierungen im Brillenglas selbst vorgenommen werden, um es auf die Trageposition in Gebrauchsstellung anzupassen.

Es sind auch kamerabasierte Vorrichtungen bekannt, bei denen der Inhalt auf einem Bildschirm in Abhängigkeit von der Lage und/oder der Kopfposition des Benutzers vor dem Bildschirm variiert. Eine beispielhafte Vorrichtung, die zur Prüfung der Sehschärfe eines Benutzers ausgelegt ist, bei der die Größe der Sehtestzeichen in Abhängigkeit von der Position des Benutzers variieren kann, ist in DE 10 2014 009 459 offenbart.

In den obigen kamerabasierten Vorrichtungen ist es häufig wichtig, Informationen über die Lage eines von der Kamera aufgenommenen Objekts, z.B. eines Auges eines Benutzers, relativ zur Kamera zu erhalten, insbesondere Informationen über den Abstand des Objekts zur Kamera.

Die Druckschrift DE 10 2005 038218 A1 beschreibt ein System zum Bestimmen des Abstands zwischen dem Auge eines Benutzers und einem Messgerät anhand von Lichtreflexionen auf der Hornhaut des Auges. Ferner kann der Krümmungsradius der Hornhaut im Scheitelpunkt des Auges oder der Krümmungsradius im Pupillenmitte (K-Wert des Auges) bestimmt werden.

Die Druckschrift WO 2008/009355 beschreibt eine Vorrichtung und ein Verfahren zum Bestimmen unterschiedlicher Parameter der Trägerposition einer Brille.

Die Druckschrift WO 2014/103646 beschreibt eine ophthalmologische Vorrichtung zum Durchführen von Augenuntersuchungen. Die Vorrichtung umfasst ein optisches Untersuchungssystem (z.B. OCT), mindestens zwei Kameras, die die das anteriore Segment eines Auges aus unterschiedlichen Richtungen gleichzeitig fotografieren, und eine Antriebsvorrichtung, die das optische Untersuchungssystem bewegt. Die dreidimensionale Position des Auges wird anhand der von den Kameras aufgenommenen Bilder ermittelt und auf Basis von vorab gespeicherten Korrekturinformationen korrigiert. Die korrigierten Positionsdaten steuern die Bewegung des optischen Untersuchungssystems durch die Antriebsvorrichtung.

Eine Aufgabe der Erfindung ist es, eine verbesserte und einfache Möglichkeit zum Ermitteln des Abstands eines Auges oder beider Augen eines Benutzers von einer Bildaufnahmeeinrichtung bereitzustellen. Eine weitere Aufgabe der Erfindung ist es, eine verbesserte und einfache Möglichkeit zum Ermitteln optischer Parameter eines Benutzers bereitzustellen. Diese und andere Aufgaben der Erfindung werden durch die Gegenstände der unabhängigen Ansprüche gelöst.

Ein erster Aspekt betrifft eine Vorrichtung mit einer Bildaufnahmeeinrichtung und einer Messeinrichtung zum Bestimmen des Abstands zumindest eines Auges eines Benutzers von der Bildaufnahmeeinrichtung (Abstand Auge-Bildaufnahmeeinrichtung). Die Messeinrichtung umfasst eine Beleuchtungseinrichtung, welche in einer vorgegebenen relativen Position zur Bildaufnahmeeinrichtung angeordnet ist, und welche ausgelegt und eingerichtet ist, zumindest eine Lichtreflexion mit einem im Wesentlichen linienförmigen bzw. linearen bzw. ausgedehnten Abschnitt auf der Hornhaut (Cornea) des Auges des Benutzers zu erzeugen. Dementsprechend umfasst die Beleuchtungseinrichtung eine Lichtquelle, die die Lichtreflexion auf der Hornhaut des untersuchten Auges erzeugt. Die Lichtquelle kann mehrere Leuchtpunkte aufweisen, die in einem bestimmten Muster angeordnet sind. Insbesondere kann die Lichtquelle einen im Wesentlichen linienförmigen bzw. ausgedehnten Abschnitt aufweisen. Die Form bzw. die Struktur der Lichtquelle kann vorbekannt sein, insbesondere kann der Abstand zwischen zumindest zwei vorbestimmten bzw. vorgegebenen Punkten der Lichtquelle und die Form und/oder die Position zumindest eines im Wesentlichen linienförmigen Abschnitts der Lichtquelle vorbekannt sein. Die Lichtquelle kann eine strukturierte Lichtquelle sein mit mehreren einzelnen Lichtquellen. Der im Wesentlichen linienförmige Abschnitt der Lichtquelle kann z.B. ein gerader Abschnitt sein.

Die Bildaufnahmeeinrichtung ist ausgelegt und eingerichtet, zumindest ein Bild der spekularen bzw. spiegelnden Lichtreflexion bzw. der Spiegelung der Lichtquelle auf der Hornhaut des untersuchten Auges des Benutzers aufzunehmen. Die Bildaufnahmeeinrichtung kann eine oder mehrere Kameras umfassen, die auf die Augenpartie des Benutzers gerichtet sind und zumindest ein Bild aufnehmen können, in dem die spekulare bzw. spiegelnde Lichtreflexion auf der Hornhaut des zumindest einen Auges sichtbar ist.

Die Messeinrichtung umfasst ferner eine Abstandsbestimmungseinrichtung zum Bestimmen des Abstands Auge-Bildaufnahmeeinrichtung, die in einer Datenverarbeitungseinrichtung implementiert sein kann. Die Abstandsbestimmungseinrichtung ist ausgelegt, das zumindest eine von der Bildaufnahmeeinrichtung aufgenommene Bild zu bearbeiten und Daten bezüglich des Abstands zwischen den zwei vorbestimmten bzw. vorbekannten Punkten in der Lichtreflexion und bezüglich der Krümmung des zumindest einen linienförmigen Abschnitts der Lichtreflexion im zumindest einen aufgenommenen Bild zu ermitteln. Ferner ist die Abstandsbestimmungseinrichtung ausgelegt, anhand der ermittelten Daten bezüglich des Abstands zwischen den zwei vorbestimmten Punkten der Lichtreflexion und einer Krümmung des zumindest einen linienförmigen Abschnitts der Lichtreflexion, den Abstand des Auges zur Bildaufnahmeeinrichtung zu ermitteln. Die Abstandsbestimmungseinrichtung kann einen Prozessor aufweisen oder als ein entsprechendes Modul eines Prozessors oder Computers realisiert sein. Die Abstandsbestimmungseinrichtung kann mittels des entsprechend programmierten Mikroprozessors eines mobilen Computers (wie z.B. Notebooks, Laptops, Tablets, Smartphones, etc.) realisiert werden.

Insbesondere kann bei bekannter Form und Abmessung der Lichtquelle der Abstand des Auges zur Bildaufnahmeeinrichtung anhand der spekularen Lichtreflexion in zumindest einem von der Bildaufnahmeeinrichtung aufgenommenen Bild ermittelt werden. So ist die Form der spekularen Lichtreflexion auf der Hornhaut im aufgenommenen Bild vorrangig von der Geometrie der Hornhaut des untersuchten Auges abhängig. Die Größe der Lichtreflexion ist vorrangig von der Geometrie des Messsystems und insbesondere von dem Abstand zwischen der Bildaufnahmeeinrichtung und des Auges abhängig. Folglich kann anhand des gemessenen Abstands zwischen zwei vorbekannten bzw. vorbestimmten Punkten der Lichtreflexion im aufgenommenen Bild der Abstand zwischen der Bildaufnahmeeinrichtung und dem untersuchten Auge ermittelt werden. Die vorbekannten bzw. vorbestimmten Punkte der Lichtreflexion können z.B. die Endpunkte des linienförmigen Abschnitts der Lichtreflexion sein, so dass anhand der im Bild gemessenen Länge des linienförmigen Abschnitts Informationen über den Abstand Auge-Bildaufnahmeeinrichtung gewonnen werden können. Die vorbestimmten Punkte können auch andere Punkte der Lichtquelle sein, deren ursprüngliche Position in der Lichtquelle bekannt ist.

Um die Messgenauigkeit zu erhöhen wird ferner vorgeschlagen, bei der Bestimmung des Abstands Auge-Bildaufnahmeeinrichtung die Abhängigkeit der Krümmung des linienförmigen Abschnitts der Lichtreflexion im aufgenommenen Bild von dem Abstand zwischen der Bildaufnahmeeinrichtung und dem Auge ebenfalls zu berücksichtigen. Der Abstand des Auges zur Bildaufnahmeeinrichtung wird somit anhand von Daten bezüglich des Abstands zwischen zwei vorbekannten Punkten der Lichtreflexion und der Krümmung des zumindest einen linienförmigen Abschnitts der Lichtquelle im aufgenommenen Bild ermittelt. Somit kann eine einfache und genaue Messeinrichtung zum Bestimmen des Abstands Auge-Bildaufnahmeeinrichtung realisiert werden, die insbesondere zur mobilen Anwendung geeignet ist.

Die Beleuchtungseinrichtung kann ausgelegt sein, je eine Lichtreflexion auf der Hornhaut jedes der beiden Augen zu erzeugen. Die Bildaufnahmeeinrichtung kann ausgelegt sein, zumindest ein Bild der beiden Augen aufzunehmen. Die Abstandsbestimmungseinrichtung kann ausgelegt sein, den Abstand Auge-Bildaufnahmeeinrichtung getrennt für beide Augen des Benutzers zu ermitteln. Anhand der ermittelten Abstände Auge-Bildaufnahmeeinrichtung kann ein Mittelwert gebildet werden. Der ermittelte Mittelwert kann als Abstand Auge-Bildaufnahmeeinrichtung ausgegeben werden. Daneben kann aus den Abständen der beiden Augen zur Bildaufnahmeeinrichtung auch ein Winkel zwischen der Bildebene der Bildaufnahmeeinrichtung und der Verbindungslinie zwischen den Augen ermittelt werden, aus dem die Kopfdrehung ermittelt werden kann.

Die Abstandsbestimmungseinrichtung kann dazu ausgebildet und vorgesehen sein, die zwei vorbestimmten Punkte der Lichtreflexion und den zumindest einen linienförmigen Abschnitt der Lichtreflexion automatisch und/oder manuell im aufgenommenen Bild zu lokalisieren. Dabei beinhaltet diese Lokalisierung Ortsinformationen in Form von Koordinaten und/oder Pixeln, aus denen die Abstandsbestimmungseinrichtung die Daten bezüglichen des Abstands der zwei (oder mehr) vorbestimmten Punkte und der Krümmung des linienförmigen Abschnitts der Lichtreflexion ermittelt. Dabei kann die Lokalisierung in jeder Koordinatenrichtung der zweidimensionalen Aufnahme auf zwei Pixel genau erfolgen, bevorzugt auf ein Pixel genau, besonders bevorzugt Subpixel genau, also z.B. auf 1/10 Pixel genau. Dies kann z.B. durch Anlegen und Auswerten einer Gaussverteilung bezüglich der Lichthelligkeit der Pixel im aufgenommenen Bild erfolgen.

Der Abstand des Auges zur Bildaufnahmeeinrichtung kann z.B. unter Verwendung von Informationen bezüglich der (festen) relativen Anordnung der Beleuchtungseinrichtung zur Bildaufnahmeeinrichtung und gegebenenfalls weiterer Informationen bezüglich des optischen Systems der Bildaufnahmeeinrichtung (wie z.B. Vergrößerung, etc.) ermittelt werden. Diese Informationen können als Kalibrierungsdaten in geeigneter Form in einer Kalibrierungsdatenspeichereinrichtung gespeichert sein. Die Kalibrierungsdatenspeichereinrichtung kann Bestandteil der Abstandsbestimmungseinrichtung sein.

Die Kalibrierungsdaten, die bei der Bestimmung des Abstands Auge-Bildaufnahmeeinrichtung berücksichtigt werden, können z.B. Daten bezüglich der Zuordnung von Abständen zwischen den zwei vorbestimmten Punkten der Lichtreflexion und von Krümmungen des zumindest einen linienförmigen Abschnitts der Lichtreflexion im aufgenommenen Bild zu bestimmten bzw. vorabbestimmten Abständen von Messobjekten zur Bildaufnahmeeinrichtung enthalten. Die Kalibrierungsdaten können anhand von vorbekannten Messobjekten, die in vorbekannten Abständen zur Bildaufnahmeeinrichtung angeordnet sind, gewonnen werden. Die Messobjekte können Objekte mit einer reflektierenden sphärischen Oberfläche mit vorbekanntem Krümmungsradius sein, wie z.B. sphärische Linsen oder Kugeln (z.B. aus Metall). Vorzugsweise ist der Krümmungsradius der sphärischen Fläche zwischen 7 mm und 10 mm. Dieser Bereich entspricht der Krümmung der menschlichen Hornhaut.

Die Kalibrierungsdaten können mittels einer Interpolation mehrerer Datenpunkten gewonnen werden. Die Datenpunkte können z.B. Messwerte für den Abstand zwischen zwei vorgegebenen Punkten der Lichtreflexion und die Krümmung der Reflexion des linienförmigen Abschnitts der Lichtreflexion in einem Bild enthalten, das durch die Kamera der zu kalibrierenden Messvorrichtung aufgenommen wurde. Die Messwerte können für mehrere unterschiedlich gekrümmte Messobjekte mit bekannten Krümmungen und für mehrere unterschiedliche, vorbekannte Abstände Messobjekt-Bildaufnahmeeinrichtung gewonnen werden. Die Kalibrierungsdaten können in geeigneter Form gespeichert werden, wie z.B. tabellarisch, graphisch, funktional, etc.

Die in der Beleuchtungseinrichtung angeordnete Lichtquelle kann eine diffus strahlende Lichtquelle sein. Das von der Lichtquelle ausgestrahlte Beleuchtungslicht weist bevorzugt eine Lichtwellenlänge auf, die in den Bilddaten schnell und sicher erkannt werden kann, z.B. automatisch über eine computer-gesteuerte Auswertung. Die Lichtquelle kann z.B. zumindest eine Leuchtstoffröhre, LED, etc. umfassen. Als Lichtquelle kann ebenfalls ein auf einem Bildschirm (wie z.B. der Bildschirm eines mobilen Computers) angezeigtes Muster dienen.

Grundsätzlich kann die Form der Lichtquelle und somit der von der Lichtquelle erzeugten Lichtreflexion unterschiedlich bzw. weitgehend frei sein, da es ausreichend ist, wenn die Lichtquelle zumindest einen im Wesentlichen linienförmigen, insbesondere einen geraden Abschnitt aufweist. So kann die Lichtquelle ein Buchstabe, ein Symbol oder ein Logo sein. In einem Beispiel besteht die Lichtquelle aus dem im Wesentlichen geraden Abschnitt. So kann die Lichtquelle die Form eines Lichtbalkens aufweisen. Die zwei vorbestimmten Punkte, zwischen denen der Abstand im Bild der spekularen Lichtreflexion auf der Hornhaut gemessen wird, können die Endpunkte des Lichtbalkens sein. In diesem Fall entspricht der gemessene Abstand im Bild der Lichtreflexion der Länge des linienförmigen Abschnitts bzw. des Lichtbalkens. Dies ermöglicht eine einfache Ausgestaltung der Beleuchtungseinrichtung.

Um die Messgenauigkeit zu erhöhen, kann die Lichtquelle ferner zumindest zwei punkt- bzw. kreisförmige Abschnitte bzw. Bereiche aufweisen, welche die Punkte markieren, dessen Abstand im aufgenommenen Bild der spekularen Lichtreflexion auf der Hornhaut gemessen wird. So kann die Lichtquelle z.B. die Form eines Lichtbalkens mit zwei im Wesentlichen punkt- bzw. kreisförmigen Leuchtflecken an beiden Enden des Lichtbalkens aufweisen. Die Leuchtflecken können auch oberhalb und unterhalb des Lichtbalkens in einem vorgegebenen Abstand vom Lichtbalken angeordnet werden.

Die Lichtquelle und somit die von der Lichtquelle erzeugte spekulare Lichtreflexion kann ebenfalls mehrere im Wesentlichen lineare bzw. ausgedehnte und insbesondere gerade Abschnitte und/oder Abschnitte mit komplizierteren Formen aufweisen. Daten über die Form und/oder Anordnung dieser Abschnitte im aufgenommenen Bild können bei der Bestimmung des Abstands Auge-Bildaufnahmeeinrichtung verwendet werden, um die Messgenauigkeit zu erhöhen.

Anhand des ermittelten Abstands Auge-Bildaufnahmeeinrichtung lassen sich weitere Informationen und/oder Parameter ermitteln. Die Vorrichtung kann dementsprechend eine Datenverarbeitungseinrichtung umfassen, die ausgelegt ist, die weiteren Informationen und/oder Parameter unter Berücksichtigung des ermittelten Abstands Auge-Bildaufnahmeeinrichtung zu ermitteln.

So kann die Vorrichtung eine Skalierungsfaktorbestimmungseinrichtung umfassen, welche ausgelegt ist, anhand des ermittelten Abstands zumindest eines der Augen zur Bildaufnahmeeinrichtung einen Skalierungsfaktor zu ermitteln zum Umrechnen einer, in einem von der Bildaufnahmeeinrichtung aufgenommenen Bild gemessenen Distanz in eine tatsächliche Distanz zu ermitteln. Die im Bild gemessenen Distanzen können z.B. in Pixel angegeben werden.

Der Skalierungsfaktor kann die Umrechnung von in Pixeln gemessenen Distanzen im Bild in tatsächliche Distanzen wiedergeben. Es ist ferner möglich, für jedes Auge des Benutzers einen separaten Skalierungsfaktor zu ermitteln. Sind die ermittelten Abstände Auge-Bildaufnahmeeinrichtung für beide Augen unterschiedlich, kann der Berechnung des Skalierungsfaktors einen Mittelwert der beiden Abstände zugrunde gelegt werden.

Das von der Bildaufnahmeeinrichtung aufgenommene Bild kann z.B. eine Aufnahme beider Pupillen des Benutzers, vorzugsweise mit der jeweiligen spekularen Lichtreflexion auf der Hornhaut, umfassen. Anhand des aufgenommenen Bilds kann die Position der beiden Pupillen der Augen des Benutzers und die Distanz der beiden Pupillen im aufgenommenen Bild (z.B. in Pixel) ermittelt werden. Durch die Bestimmung der Distanz der beiden Pupillen im Bild und unter Berücksichtigung der Skalierungsfaktoren für beide Augen kann über eine Mittelung der tatsächliche Abstand zwischen den beiden Augen bzw. zwischen den beiden Pupillen in der Aufnahmesituation ermittelt werden. Bei einem bekannten Fixierpunkt relativ zur Bildaufnahmeeinrichtung bzw. zur Kamera kann die Pupillendistanz mit Hilfe eines Augenmodels für den Blick nach Unendlich umgerechnet werden. In einem Beispiel kann der Fixierpunkt das Kameraobjektiv bzw. ein Punkt des Kameraobjektivs sein.

Über den Skalierungsfaktor kann z.B. der Pupillendurchmesser eines Auges ermittelt werden, indem z.B. aus einem im aufgenommenen Bild ermittelten Pupillendurchmesser mittels des Skalierungsfaktors der tatsächliche Pupillendurchmesser berechnet wird.

Ferner ist es möglich, einen (z.B. zweiten) Skalierungsfaktor für die Fassungsebene einer vom Benutzer während der Bildaufnahme getragenen Brillenfassung oder Brille zu ermitteln. Die Fassungsebene ist die Ebene, die durch zueinander vertikale Mittellinien der die rechte und linke Scheibenebene der Brillenfassung festlegenden Kastensysteme gebildet ist (siehe z.B. DIN 58 208).

Der Berechnung des Skalierungsfaktors für die Fassungsebene einer vom Benutzer getragenen Brillenfassung oder Brille kann ein Hornhautscheitelabstand zugrunde gelegt werden. Der Hornhautscheitelabstand kann z.B. ein angenommener, durchschnittlicher oder normierter Hornhautscheitelabstand sein. Der Hornhautscheitelabstand kann auch ein zuvor durch anderweitige Messung bestimmter, für den Benutzer individueller Hornhautscheitelabstand sein. Der Skalierungsfaktor für die Fassungsebene einer vom Benutzer getragenen Brillenfassung ermöglicht die Umrechnung der im Bild des Benutzers mit der Brillenfassung gemessenen Distanzen in entsprechende "tatsächliche" Distanzen in der Fassungsebene. Der Skalierungsfaktor für die Fassungsebene kann zur Bestimmung weiterer Parameter wie z.B. Scheibenlänge, Scheibenhöhe, Zentrierhöhe, Abstand zwischen den Gläsern, Glasdurchmesser, etc. verwendet werden. Weitere Korrekturen sind durch vorherige zusätzliche Eingaben weiterer Parameter, wie z.B. Vorneigung, Fassungsscheibenwinkel, etc. möglich. Diese Parameter können durchschnittliche Parameter sein. Die Parameter können auch anderweitig, z.B. durch bereits bekannte Messverfahren, ermittelt werden. Die Vorrichtung kann entsprechende Parametererfassungsmittel zum Erfassen zumindest eines Parameters der Gebrauchsstellung einer vom Benutzer getragenen Brille oder Brillenfassung, wie z.B. Vorneigung, Fassungsscheibenwinkel und/oder Hornhautscheitelabstand, umfassen.

Die Vorrichtung kann eine Parameterbestimmungseinrichtung umfassen, welche ausgelegt ist, zumindest einen optischen Parameter zumindest eines der Augen des Benutzers und/oder einen Parameter der Gebrauchsstellung einer Brille oder Brillenfassung vor den Augen des Benutzers unter Berücksichtigung des ermittelten Abstandes des Auges zur Bildaufnahmeeinrichtung zu ermitteln. Der zumindest eine optische Parameter kann insbesondere anhand der im aufgenommenen Bild gemessenen Distanzen und unter Berücksichtigung der zuvor ermittelten Skalierungsfaktoren ermittelt werden.

Der zumindest eine optische Parameter des Benutzers kann insbesondere die monokulare Pupillendistanz und/oder die Pupillendistanz und/oder die Scheibenlänge einer vom Benutzer getragenen Brille oder Brillenfassung und/oder die Scheibenhöhe einer vom Benutzer getragenen Brille oder Brillenfassung und/oder die Zentrierhöhe einer vom Benutzer getragenen Brille oder Brillenfassung und/oder der Abstand zwischen den Gläsern einer vom Benutzer getragenen Brille oder zwischen den Gläserringen einer vom Benutzer getragenen Brillenfassung und/oder der Durchmesser der Gläser einer vom Benutzer getragenen Brille oder der Gläserringe einer vom Benutzer getragenen Brillenfassung und/oder der Pupillendurchmesser eines oder beider Augen des Benutzers sein.

Die obigen und weitere Parameter des Benutzers und/oder der Gebrauchsstellung einer Brille oder Brillenfassung vor den Augen des Benutzers sind beispielsweise in DIN EN ISO 8624 und/oder DIN EN ISO 1366 und/oder DIN 58208 und/oder DIN 5340 beschrieben. Ferner wird hinsichtlich der Definition der Parameter der Augen eines Benutzers oder der Gebrauchsstellung einer Brille oder Brillenfassung vor den Augen des Benutzers auf das Buch "Die Optik des Auges und der Sehhilfen" von Dr. Roland Enders, 1995, Optische Fachveröffentlichung GmbH, Heidelberg verwiesen. Die Normen sowie das genannte Buch stellen für die Begriffsdefinitionen insoweit einen integralen Offenbarungsbestandteil der vorliegenden Anmeldung dar.

Die Bildaufnahmeeinrichtung kann eine digitale Kamera (z.B. eine CCD Kamera) umfassen, die auf das Auge oder die Augen des Benutzers gerichtet ist. Ferner kann die Bildaufnahmeeinrichtung weitere optische Komponenten (z.B. Linsen, Spiegel, Strahlteiler, etc.) umfassen, um einen geeigneten Strahlengang, eine geeignete Vergrößerung, etc. zu erzielen.

Um eine Art Stereokamerasystem zu realisieren, kann die Bildaufnahmeeinrichtung ausgelegt und eingerichtet sein, Bilder der spekularen bzw. spiegelnden Reflexion der zumindest einen Lichtquelle im Bereich der Hornhaut des Auges des Benutzers aus zumindest zwei unterschiedlichen Blickwinkeln bzw. Aufnahmepositionen aufzunehmen, wobei pro Blickwinkel bzw. pro Aufnahmeposition zumindest ein Bild aufgenommen wird. Wenn die Bildaufnahmeeinrichtung eine Kamera umfasst, kann diese drehbar bzw. schwenkbar angeordnet sein, um Bilder aus zumindest zwei unterschiedlichen Perspektiven aufzunehmen. Die Bildaufnahmeeinrichtung kann auch zumindest zwei Kameras umfassen, die Bilder aus unterschiedlichen Blickwinkeln bzw. aus unterschiedlichen Aufnahmepositionen im Wesentlichen simultan aufnehmen.

Anhand der aufgenommenen Bilder kann der Abstand Auge-Bildaufnahmeeinrichtung für jeden der Blickwinkel bzw. für jede der Aufnahmepositionen ermittelt werden. So kann die Abstandsbestimmungseinrichtung ausgelegt sein, für jeden der Blickwinkel bzw. für jede der Aufnahmepositionen:

Daten bezüglich des Abstands zwischen den zwei vorbestimmten Punkten der Lichtreflexion und der Krümmung des zumindest einen linienförmigen Abschnitts der Lichtreflexion in dem zumindest einen aus diesem Blickwinkel bzw. aus dieser Aufnahmeposition aufgenommenen Bild zu ermitteln; und
anhand der ermittelten Daten bezüglich des Abstands zwischen den zwei vorbestimmten Punkten der Lichtreflexion und der Krümmung des zumindest einen linienförmigen Abschnitts der Lichtreflexion den Abstand des Auges zur Bildaufnahmeeinrichtung bei diesem Blickwinkel bzw. bei dieser Aufnahmeposition zu ermitteln.

Über die ermittelten Abstände des Auges bzw. der Augen von der Bildaufnahmeeinrichtung bei unterschiedlichen Blickwinkeln kann eine Änderung der Aufnahmeposition ermittelt werden. Hierzu können zusätzlich die Aufnahme eines statischen Objekts und die Berücksichtigung des Winkels von der optischen Achse der Bildaufnahmeeinrichtung zur Horizontalen verwendet werden. Die jeweilige Aufnahmeposition kann aus den Abständen Auge-Bildaufnahmeeinrichtung in einer horizontalen Projektionsebene ermittelt werden. Mit dieser Information kann aus den zumindest zwei aus unterschiedlichen Blickwinkeln bzw. Aufnahmepositionen aufgenommenen Bildern ein Stereokamerasystem bzw. ein stereoskopisches Messsystem realisiert werden. Die horizontale Ebene kann die horizontale Ebene im Bezugssystem des Benutzers und/oder im Bezugssystem der Erde angeordnet sein und durch den Mittelpunkt einer Pupille verlaufen. Dies ist insbesondere dann der Fall, wenn die beiden Augen des Benutzers beispielsweise in unterschiedlicher Höhe (im Bezugssystem der Erde) angeordnet sind.

Die Vorrichtung kann ferner eine 3D-Rekonstruktionseinrichtung umfassen, welche ausgelegt ist, anhand der aus unterschiedlichen Aufnahmepositionen aufgenommenen Bilder und der ermittelten Abstände des zumindest einen Auges des Benutzers zur Bildaufnahmeeinrichtung für jede der Aufnahmepositionen Ortsinformationen im dreidimensionalen Raum für zumindest einen Punkt und/oder einen Bereich des zumindest einen Auges des Benutzers und/oder einer vor dem Auge angeordneten Brille oder Brillenfassung zu ermitteln. So können z.B. dreidimensionale Ortsinformationen für zumindest einen der folgenden Punkte ermittelt werden:

- für einen Schnittpunkt einer im Bezugssystem des Benutzers horizontalen Ebene mit den Brillenglasrändern und/oder den Brillenfassungsrändern der Brille, wobei die horizontale Ebene des Benutzers beide Pupillen des Benutzers schneidet und parallel zu einer vorbestimmten Nullblicklinie des Benutzers verläuft;
- für einen Schnittpunkt einer im Bezugssystem des Benutzers vertikalen Ebene mit den Brillenglasrändern und/oder den Brillenfassungsrändern der Brille, wobei die vertikale Ebene des Benutzers senkrecht zur horizontalen Ebene des Benutzers und parallel zu der vorbestimmten Nullblicklinie des Benutzers verläuft und eine Pupille des Benutzers schneidet;
- für zumindest einen Pupillenmittelpunkt;
- für die Begrenzungen zumindest eines Brillenglases des Benutzers nach einer Bemaßung im Kastenmaß;
- für den Brillenmittelpunkt der Brillenfassung der Brille.

Unter einer Bemaßung im Kastenmaß wird im Sinne dieser Erfindung das Maßsystem verstanden, wie es in einschlägigen Normen, beispielsweise in der DIN EN ISO 8624 und/oder der DIN EN ISO 13666 DIN und/oder der DIN 58208 und/oder der DIN 5340 beschrieben wird.

Anhand der ermittelten dreidimensionalen Ortsinformationen können Zentrierdaten und individuelle Parameter des Benutzers ermittelt werden, wie z.B. die zuvor genannten optischen Parameter des Benutzers. Beispielhafte Vorrichtungen und Verfahren zum Bestimmen von Zentrierdaten und individuellen Parametern des Benutzers im dreidimensionalen Raum sind z.B. in den Patentanmeldungen DE 10 2005 003 699 A1 und DE 10 2014 015 671 offenbart.

Die Ermittlung kann eine Bestimmung von korrespondierenden Punkten in den erfassten Bildern aus unterschiedlichen Aufnahmepositionen bzw. aus unterschiedlichen Blickwinkel umfassen. Die korrespondierenden Punkte können mittels einer manuellen Auswertung ermittelt werden. Die korrespondierenden Punkte können auch automatisch, z.B. anhand des in DE 10 2014 015 671 beschriebenen Verfahrens ermittelt werden.

Der ermittelte Abstand Auge-Bildaufnahmevorrichtung kann auch für weitere Anwendungen verwendet werden. So kann der Abstand Auge-Bildaufnahmevorrichtung als Parameter bei einer S ehaufgabe, welche auf einem Bildschirm (z.B. auf dem Bildschirm eines Laptops, Notebooks, Tablets, Smartphones, etc.) in variabler Entfernung dargestellt wird, verwendet werden. Im Allgemeinen kann der Abstand Auge-Bildaufnahmevorrichtung als Parameter bei einer variablen Darstellung beliebiger Inhalte abhängig von der Kopfposition des Betrachters vor einem Bildschirm, etc. verwendet werden.

Die Vorrichtung kann dementsprechend eine Vorrichtung zum Anzeigen beliebiger Daten bzw. Inhalte auf einem Bildschirm sein, wobei der Bildschirm in einer vorgegebenen bzw. bekannten relativen Position zur Bildaufnahmeinrichtung angeordnet ist. Die Vorrichtung kann eine Bildschirminhaltermittlungseinrichtung umfassen, welche ausgelegt ist, den Abstand zwischen dem Bildschirm und einem Benutzer bzw. einem Betrachter anhand des ermittelten Abstands zwischen dem zumindest einen Auge des Benutzers und der Bildaufnahmeeinrichtung zu ermitteln. Anhand des ermittelten Abstands "Bildschirm-Benutzer" kann der auf dem Bildschirm anzuzeigende bzw. angezeigte Bildschirminhalt in Abhängigkeit von dem ermittelten Abstand zwischen dem Benutzer und dem Bildschirm verändert werden. So können z.B. die Größe und/oder die Position der auf dem Bildschirm angezeigten Bilder in Abhängigkeit von dem ermittelten Abstand Auge-Bildaufnahmeeinrichtung variiert werden.

Die Vorrichtung kann auch eine Vorrichtung zur Sehschärfebestimmung mit einem Bildschirm sein, auf welchem unterschiedliche Sehzeichen (so genannte Optotypen) zum Durchführen eines Sehtests angezeigt werden. Der Abstand des Benutzers von dem Bildschirm kann in Abhängigkeit von dem ermittelten Abstand Auge-Bildaufnahmevorrichtung ermittelt werden (bei bekannter relativer Anordnung des Bildschirms zur Bildaufnahmeeinrichtung). Die individuelle Sollgröße eines auf dem Bildschirm gezeigten Sehzeichens kann in Abhängigkeit von dem ermittelten Abstand Auge-Bildschirm ermittelt werden. Eine beispielhafte Vorrichtung zur Sehschärfebestimmung mit variabler Größe der Sehzeichen ist z.B. in der Anmeldung DE 10 2014 009 459 offenbart.

Die Vorrichtung kann als eine tragbare, mobile Vorrichtung ausgebildet sein. Die Vorrichtung kann dabei in der Hand einer Bedienperson getragen und bedient werden. Für den mobilen Einsatz kann zum Beispiel das Objektiv der Bildaufnahmeeinrichtung als ein Fixierobjekt für den Benutzer verwendet werden, der in einer Nullblickrichtung auf das Objektiv blickt.

Die Vorrichtung kann insbesondere in einen mobilen Computer (wie z.B. Notebook, Tablet, Smartphone, etc.) integriert sein. Dabei dient die in dem mobilen Computer integrierte Kamera als die Bildaufnahmeeinrichtung. Die oben beschriebenen Berechnungen des Abstands Auge-Bildaufnahmeeinrichtung, des Skalierungsfaktors, der optischen Parameter, der Ortsdaten im dreidimensionalen Raum, etc. können mittels des Prozessors des mobilen Computers durchgeführt werden. Die Beleuchtungseinrichtung kann zumindest eine Lichtquelle (z.B. eine Linienlichtquelle) umfassen, die am Gehäuse des mobilen Computers befestigt ist und/oder in dem Gehäuse integriert ist. Es ist auch möglich, den Bildschirm des mobilen Computers als Lichtquelle zu benutzen. Eine Kombination unterschiedlicher Lichtquellen (z.B. Bildschirm mit zusätzlicher Lichtquelle(n)) ist auch möglich.

Die Vorrichtung kann ferner einen Speicher zum Speichern des ermittelten Abstands Auge-Bildaufnahmeeinrichtung und gegebenenfalls weiterer Daten (wie z.B. die ermittelten optischen Parameter) und/oder eine Datenausgabeeinrichtung (umfassend z.B. einen Bildschirm) zur Ausgabe des ermittelten Abstands Auge-Bildaufnahmeeinrichtung und gegebenenfalls weiterer Daten umfassen. Die Abstandsbestimmungseinrichtung, die Kalibrierungsdatenspeichereinrichtung, die Skalierungsfaktorbestimmungseinrichtung, die Parametererfassungsmittel, die Parameterbestimmungseinrichtung, die 3D-Rekonstruktionseinrichtung und/oder der Speicher können in einer Datenverarbeitungseinrichtung umfassend einen oder mehrere Prozessoren bzw. einen oder mehrere Computer implementiert werden.

Ein zweiter Aspekt der Erfindung betrifft ein Verfahren umfassend:

Erzeugen einer spekularen Lichtreflexion mit zumindest einem linienförmigen Abschnitt auf der Hornhaut eines Auges eines Benutzers, wobei die Lichtreflexion von einer Beleuchtungseinrichtung erzeugt wird, welche in einer festen relativen Position von einer Bildaufnahmeeinrichtung angeordnet ist;

Aufnehmen zumindest eines Bildes der spekularen bzw. spiegelnden Lichtreflexion im Bereich der Hornhaut des Auges des Benutzers;

Ermitteln des Abstands zwischen zwei vorbestimmten bzw. vorgegebenen Punkten der Lichtreflexion und der Krümmung des zumindest einen linienförmigen Abschnitts der Lichtreflexion im aufgenommenen Bild; und

Ermitteln des Abstands des Auges des Benutzers zur Bildaufnahmeeinrichtung anhand der ermittelten Daten bezüglich des Abstands zwischen den zwei vorbestimmten bzw. vorgegebenen Punkten der Lichtreflexion und der Krümmung des zumindest einen linienförmigen Abschnitts der Lichtreflexion.

Wie oben beschrieben kann die Lichtreflexion von einer in der Bildaufnahmeeinrichtung angeordneten strukturierten Lichtquelle mit einer vorbekannten Struktur erzeugt werden. Die vorbekannte Struktur kann insbesondere einen vorbekannten Abstand zwischen zwei vorbestimmten Punkten und eine vorbekannte Form (z.B. eine Gerade) eines im Wesentlichen linienförmigen Abschnitts umfassen.

Das Verfahren kann ferner ein Speichern des ermittelten Abstands Auge-Bildaufnahmeeinrichtung in einer Speichervorrichtung, ein Anzeigen des ermittelten Abstands auf einem Bildschirm, eine akustische Widergabe des ermittelten Abstands, und/oder ein Übermitteln des ermittelten Abstands an andere Vorrichtungen, etc. umfassen. Das Ermitteln des Abstands zwischen den zwei vorbestimmten bzw. vorgegebenen Punkten der Lichtreflexion und der Krümmung des zumindest einen linienförmigen Abschnitts der Lichtreflexion im aufgenommenen Bild und das Ermitteln des Abstands des Auges des Benutzers zur Bildaufnahmeeinrichtung anhand der ermittelten Daten bezüglich des Abstands zwischen den zwei vorbestimmten Punkten der Lichtreflexion und der Krümmung des zumindest einen linienförmigen Abschnitts der Lichtreflexion können von einem geeignet programmierten Prozessor oder Computer ausgeführt werden.

Der ermittelte Abstand kann, wie oben beschrieben, unter Berücksichtigung von Kalibrierungsdaten ermittelt werden. Das Verfahren kann z.B. ein Erfassen von Kalibrierungsdaten (z.B. durch Auslesen von in einer Kalibrierungsdatenspeichereinrichtung gespeicherten Daten) und ein Ermitteln des Abstands des Auges zur Bildaufnahmeeinrichtung unter Berücksichtigung der Kalibrierungsdaten umfassen. Die Kalibrierungsdaten können Daten bezüglich einer Zuordnung von Abständen zwischen zwei vorbestimmten Punkten der Lichtreflexion und von Krümmungen des zumindest einen linienförmigen Abschnitts der Lichtreflexion im aufgenommenen Bild zu bestimmten Abständen des Auges zur Bildaufnahmeeinrichtung enthalten.

Wie oben beschrieben kann das Verfahren unterschiedlich ausgebildete bzw. strukturierte Lichtreflexionen verwenden. So kann die Lichtreflexion die Form eines Lichtbalkens aufweisen; und/oder zumindest zwei punkt- bzw. kreisförmige Bereiche aufweisen; und/oder mehrere linienförmige Abschnitte aufweisen.

Das Verfahren kann ferner ein Ermitteln eines Skalierungsfaktors zum Umrechnen einer in einem von der Bildaufnahmeeinrichtung aufgenommenen Bild gemessenen Distanz in eine tatsächliche Distanz und/oder ein Ermitteln eines Skalierungsfaktors für die Fassungsebene einer vom Benutzer getragenen Brillenfassung oder Brille anhand des ermittelten Abstandes des zumindest einen Auges zur Bildaufnahmeeinrichtung umfassen.

Das Verfahren kann ebenfalls ein Ermitteln zumindest eines optischen Parameters zumindest eines der Augen des Benutzers und/oder eines Parameters der Gebrauchsstellung einer Brille oder Brillenfassung vor den Augen des Benutzers unter Berücksichtigung des ermittelten Abstandes zumindest einen Auges zur Bildaufnahmeeinrichtung, wie vorstehend in Zusammenhang mit der Vorrichtung erläutert, umfassen.

Das Verfahren kann ein Aufnehmen von Bildern der spekularen bzw. spiegelnden Reflexion im Bereich der Hornhaut des Auges des Benutzers aus zumindest zwei unterschiedlichen Aufnahmepositionen umfassen. Für jede der Aufnahmepositionen können der Abstand zwischen zwei vorbestimmten Punkten der Lichtreflexion und die Krümmung des zumindest einen linienförmigen Abschnitts der Lichtreflexion in dem aus dieser Aufnahmeposition aufgenommenen Bild ermittelt werden. Anhand der ermittelten Daten bezüglich der Länge und der Krümmung des zumindest einen linienförmigen Abschnitts der Lichtreflexion kann der Abstand des Auges zur Bildaufnahmeeinrichtung bei dieser Aufnahmeposition ermittelt werden. Somit ist es möglich, stereoskopische Bilder zu erstellen bzw. zu generieren.

Insbesondere kann das Verfahren ein Ermitteln von Ortsinformationen im dreidimensionalen Raum für zumindest einen Punkt und/oder Bereich des zumindest einen Auges des Benutzers und/oder einer vor dem Auge angeordneten Brille oder Brillenfassung umfassen anhand der aufgenommenen Bilder aus unterschiedlichen Aufnahmepositionen und der ermittelten Abstände des zumindest einen Auges des Benutzers zur Bildaufnahmeeinrichtung für jede Aufnahmeposition.

Das Verfahren kann ferner ein Ermitteln des Abstands zwischen dem Benutzer und einem Bildschirm anhand des ermittelten Abstands zwischen dem zumindest einen Auge des Benutzers und der Bildaufnahmeeinrichtung umfassen, wobei der Bildschirm in einer vorgegebenen, bekannten relativen Position zur Bildaufnahmeeinrichtung angeordnet ist. Der ermittelte Abstand Auge-Bildschirm kann dem Benutzer angezeigt werden und/oder in einem Speicher gespeichert werden. Das Verfahren kann ferner ein Verändern des auf dem Bildschirm anzuzeigenden bzw. angezeigten Bildschirminhalts in Abhängigkeit von dem ermittelten Abstand des zumindest einen Auges des Benutzers zur Bildaufnahmeeinrichtung umfassen. So ist es z.B. möglich, ein flexibles Verfahren zum Bestimmen optischer Seheigenschaften bzw. optischer Korrekturwerte eines Benutzers zu realisieren.

Ein dritter Aspekt betrifft ein Computerprogrammprodukt umfassend Programmteile, welche, wenn geladen in einen Computer, ausgelegt sind, ein Verfahren gemäß dem zweiten Aspekt und insbesondere die in Zusammenhang mit dem Verfahren gemäß dem zweiten Aspekt erläuterten Verfahrensschritte auszuführen.

Insbesondere wird ein Computerprogrammprodukt umfassend Programmteile vorgeschlagen, welche, wenn geladen in einen Computer, ausgelegt sind, ein Verfahren mit den folgenden Schritten auszuführen:

Erfassen zumindest eines Bildes der spekularen Reflexion zumindest einer Lichtreflexion im Bereich der Hornhaut des Auges eines Benutzers, wobei die Lichtreflexion einen linienförmigen Abschnitt aufweist und von einer Beleuchtungseinrichtung ausgestrahlt wird, welche in einer festen relativen Position von einer Bildaufnahmeeinrichtung angeordnet ist;
Ermitteln des Abstands zwischen zwei vorbestimmten Punkten der Lichtreflexion und der Krümmung des zumindest einen linienförmigen Abschnitts der Lichtreflexion im erfassten Bild; und

Ermitteln des Abstands des Auges des Benutzers zur Bildaufnahmeeinrichtung anhand der ermittelten Daten bezüglich der Länge und der Krümmung des zumindest einen linienförmigen Abschnitts der Lichtreflexion.

Mit der Vorrichtung und dem Verfahren gemäß den obigen Aspekten der Erfindung kann der Abstand Auge-Bildaufnahmeeinrichtung einfach und genau bestimmt werden. Im Gegensatz zu Messvorrichtungen, wie z.B. Koinzidenz-Ophthalmometern, bei denen der Abstand der Lichtquelle zur Hornhaut entweder durch eine technische Vorrichtung (z.B. durch einen Kollimator beim Helmholtz-Typ) als konstant erzwungen wird oder bei ungenauer Kenntnis zu einer Messunsicherheit führt (Javal-Typ), wird im vorliegenden Aufbau der Abstand durch Messung bestimmt. Dabei ist keine Fixierung des zu vermessenden Benutzers durch eine Kopfstütze erforderlich. Vielmehr findet die Messung in natürlicher Kopf- und Körperhaltung des Benutzers statt. Die Vorrichtung und das Verfahren sind insbesondere zur mobilen Anwendung geeignet. So kann als Messgerät ein mobiler Computer mit integrierter Kamera und gegebenenfalls zusätzlicher Lichtquelle verwendet werden. Bei mobilen Computern, welche eine bildschirmseitige Kamera aufweisen, kann der Bildschirm selbst als Lichtquelle verwendet werden. Der ermittelte Abstand Auge-Bildaufnahmeeinrichtung kann zur Bestimmung weiterer optischer Parameter des Benutzers eingesetzt werden (wie z.B. Pupillendistanz, Einschleifhöhe, etc.). Es ist ferner möglich, ein verbessertes Stereokamerasystem zu realisieren. Der ermittelte Abstand Auge-Bildaufnahmeeinrichtung kann ebenfalls zur Darstellung von Inhalten abhängig von der Position, insbesondere der Kopfposition eines Betrachters relativ zu einem Bildschirm eingesetzt werden. So kann z.B. der ermittelte Abstand Auge-Bildaufnahmeeinrichtung als Parameter bei einer Sehaufgabe, die auf einem Tablet-Computer in variabler Entfernung dargestellt wird, eingesetzt werden. Weitere Anwendungen in kamera-basierten optischen Systemen sind ebenfalls denkbar.

Die Erfindung wird nachfolgend anhand beispielhafter Ausführungsformen und begleitender Zeichnungen näher erläutert. Es zeigen:

Figur 1      eine schematische Darstellung des Messprinzips des Abstands Auge-Bildaufnahmeeinrichtung unter Ausnutzung der Hornhautreflexion;

Figur 2      in einem dreidimensionalen Diagramm die Abhängigkeit des Abstands Messobjekt-Bildaufnahmeeinrichtung von der Objektkrümmung und der Größe des Reflexionsbilds;

Figur 3      eine schematische Darstellung einer Vorrichtung mit einer Kamera und einer Messeinrichtung zum Bestimmen des Abstands Auge-Kamera;

Figur 4      eine perspektivische, schematische Darstellung einer Vorrichtung mit einer Kamera und einer Messeinrichtung zum Bestimmen des Abstands Auge-Kamera;

Figur 5      eine schematische Darstellung einer Vorrichtung mit einer Kamera und einer Messeinrichtung zum Bestimmen des Abstands Auge-Kamera;

Figur 6      beispielhafte Lichtquellen;

Figur 7      eine beispielhafte Bildaufnahme eines Testobjekts (eines Glases);

Figur 8      ein beispielhaftes Intensitätsprofil in einem Schnitt durch die Lichtreflexion bei dem in Fig. 7B gezeigten Bild;

Figur 9      die Positionen der ermittelten Zentren der Intensitätsprofile in unterschiedlichen Schnitten durch die Lichtreflexion bei dem in Fig. 7B gezeigten Bild und eine angepasste quadratische Funktion;

Figur 10      Isolinien des Abstands Objekt-Kamera als Funktion der im Bild gemessenen Form der spekularen Lichtreflexion auf der Hornhaut;

Figur 11      Isolinien der Objektkrümmung als Funktion der im Bild gemessenen Form der spekularen Lichtreflexion auf der Hornhaut;

Figur 12      Bildaufnahmen von vier unterschiedlichen Testobjekten;

Figur 13A      ein beispielhaftes Bild der Augenpartie eines Benutzers;

Figur 13B      ein beispielhaftes Bild der spekularen Lichtreflexion einer linienförmigen Lichtquelle auf der Hornhaut.

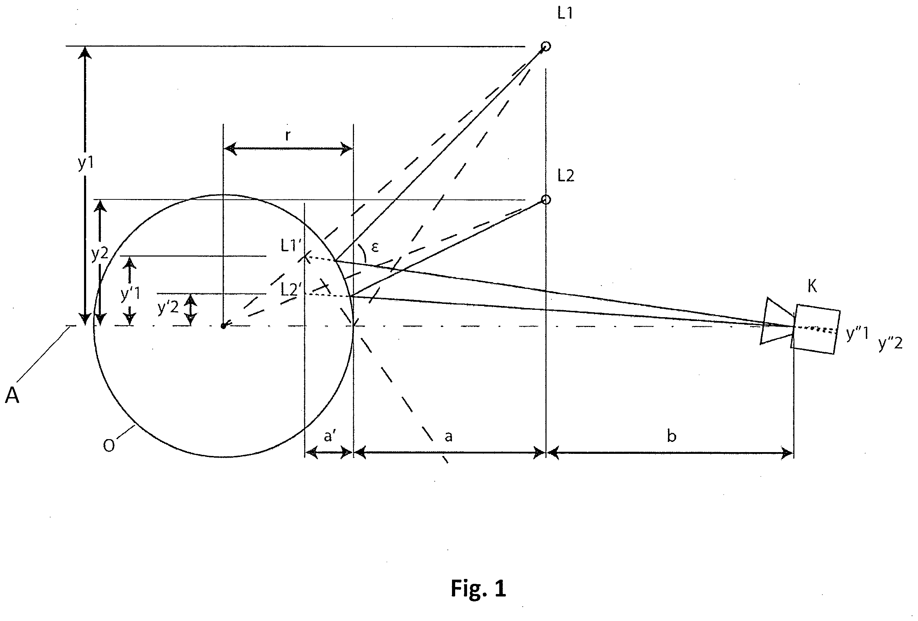

**Fig. 1** illustriert das Messprinzip eines Messsystems unter Ausnutzung der Hornhautreflexion. Das Messsystem umfasst eine Kamera (Bildaufnahmevorrichtung) K und in fester Position dazu zwei Punktlichtquellen L1 und L2. Die punktförmigen Lichtquellen L1 und L2 sind auf ein reflektierendes Objekt O gerichtet. Im Bild der Kamera K wird das Objekt O abgebildet und von jeder Lichtquelle ein spekularer bzw. spiegelnder Reflex erzeugt. Bei einem flachen Objekt, das sich in einem festen Winkel zur optischen Achse der Kamera K bewegt, ist der Abstand der Lichtreflexionen der beiden Lichtquellen abhängig von dem Abstand des Objekts O zur Kamera K. Bei einem gekrümmten Objekt hängt der Abstand der Lichtreflexionen ebenfalls von der Krümmung des Objektes ab. Bei einer konvexen Krümmung, die der Kamera K zugewandt ist, nimmt der Abstand der beiden Lichtreflexionen zueinander mit zunehmender Krümmung ab.

Das reflektierende Objekt O weist einen Krümmungsradius r auf. So wirkt z.B. die Hornhaut eines Auges wie ein sphärischer Spiegel mit einem Radius r.

Für einen sphärischen Spiegel gilt in paraxialer Näherung:

$$1/a'=1/a+2/r \qquad (1)$$

In Formel (1) bezeichnet:

a' den Abstand zwischen dem Scheitel des sphärischen Spiegels und der virtuellen Bildebene des Bilds der Lichtquellen;

a den Abstand zwischen dem Scheitel des sphärischen Spiegels und der Ebene, in der die beiden Punktlichtquellen L1 und L2 angeordnet sind; und

r den Krümmungsradius des sphärischen Spiegels.

Die Kamera K ist auf das Objekt O gerichtet. Eine Verbindungslinie zwischen einem Blendenmittelpunkt der Kamera K und einem Objektscheitelpunkt des Objekts O ist in Figur 1 strichpunktiert als Verbindungsachse A gezeigt und kann mit der optischen Achse der Kamera K zusammenfallen. In Figur 1 ist der erste Abstand y1 der ersten Lichtquelle L1 von der Verbindungsachse A gezeigt sowie der zweite Abstand der zweiten Lichtquelle L2 von der Verbindungsachse A. Die beiden Lichtquellen L1 und L2 weisen einen realen Abstand Δy=y2-y1 voneinander auf.

In der von der Kamera K angefertigten Aufnahme des Objekts O erscheint das erste virtuelle Lichtbild L1' der ersten Lichtquelle L1 unter einem ersten virtuellen Abstand y1' und das zweite virtuelle Lichtbild L2' der zweiten Lichtquelle L2 unter einem zweiten virtuellen Abstand y2' von der Verbindungsachse A. Die beiden virtuellen Lichtbilder L1' und L2' liegen gemeinsam in der virtuellen Bildebene, in der sie einen virtuellen Bildabstand Δy'=y2'-y1' voneinander aufweisen.

In der Bildebene der Kamera K erscheint das erste Lichtquellenbild unter dem ersten Bildebenenabstand y1" und das zweite Lichtquellenbild unter dem zweiten Bildebenenabstand y2" von der Verbindungsachse A. Somit weisen die beiden Lichtquellenbilder in der Bildebene der Kamera K einen Bildabstand bzw. eine Bildgröße Δy"=y2"-y1" auf.

Für die beiden Lichtquellen L1 und L2 gilt jeweils:

$$y1/(y1')=a/a' \qquad (2)$$

$$y2/(y2')=a/a' \qquad (3)$$

Von der Kamera K wird der Abstand der Bilder der beiden Lichtquellen betrachtet, also der virtuelle Bildabstand, der gegeben ist durch:

$$\Delta y'=y2'-y1' \qquad (4)$$

Ein Auflösen der Gleichungen (1) bis (3) nach a' bzw. y1' und y2' ergibt:

$$a'=(a\,r)/(2a+r) \qquad (6)$$

$$y1'=a'y1/a \qquad (7)$$

$$y2'=a'y2/a. \qquad (8)$$

Ein Einsetzen der Formel (6) in die Formeln (7) und (8) ergibt:

$$y1'=y1r/(2a+r) \qquad (9)$$

$$y2'=y2r/(2a+r) \qquad (10)$$

Für den virtuellen Bildabstand ∆y' der beiden virtuellen Lichtbilder L1' und L2' voneinander gilt somit:

$$\Delta y' = y2' - y1' = (r\,(y2 - y1))/(2a + r) \qquad (11)$$

Für eine ideale Kamera ist die Bildgröße ∆y" des virtuellen Bildabstands ∆y' der Reflexion der Lichtquellen invers proportional zum Abstand der Kamera und proportional der Größe des virtuellen Bildabstands ∆y':

$$\Delta y'' \propto \Delta y'/(a' + a + b) \qquad (12)$$

Der Abstand *b* zwischen der Kamera und der Lichtquelle ist bekannt und fest. Der Abstand a' ist im Vergleich zum Abstand *a* klein und kann vernachlässigt werden. Für *b* = 0 ergibt sich somit

$$\Delta y'' \propto r(y1 - y2)/(2a + r)a \qquad (13)$$

Bei festem realen Abstand ∆y = (y2 - y1) der Lichtquellen L1 und L2 voneinander und konstantem Radius *r* des Objekts kann somit aus dem Bild der Reflexionen der beiden Lichtquellen, insbesondere aus deren Bildabstand im Kamerabild, der Abstand *a* ermittelt werden, wenn eine kalibrierte Kamera (z.B. eine Kamera mit fixierter Optik, insbesondere mit einem konstanten Vergrößerungsfaktor) verwendet wird.

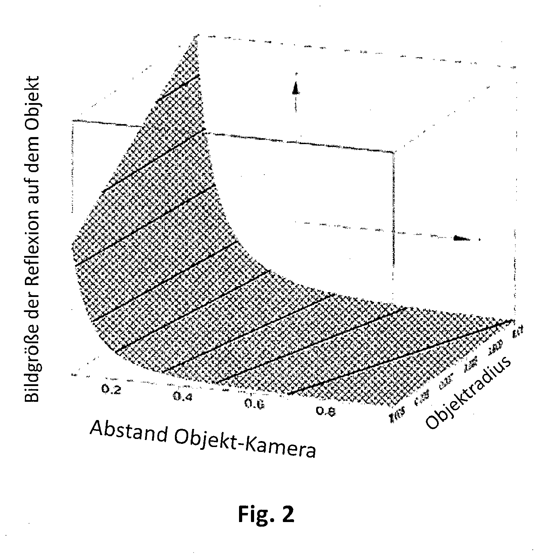

Die erhaltene Abhängigkeit des Abstands *a* von der Objektkrümmung und der Größe des Reflexionsbilds für einen festen realen Abstand ∆y = (y2 - y1) der beiden Lichtquellen voneinander ist in **Fig. 2** dargestellt.

Das obige Messprinzip lässt sich ebenfalls auf Linienlichtquellen anwenden, d.h. Lichtquellen, welche ausgedehnte, linienförmige und insbesondere gerade Abschnitte aufweisen. So kann z.B. anhand der Länge und der Krümmung der spekularen Reflexion der Linienlichtquelle auf der Hornhaut des Auges oder auf dem gekrümmten Testobjekt der Abstand zwischen dem Auge bzw. dem Testobjekt und der Kamera ermittelt werden.

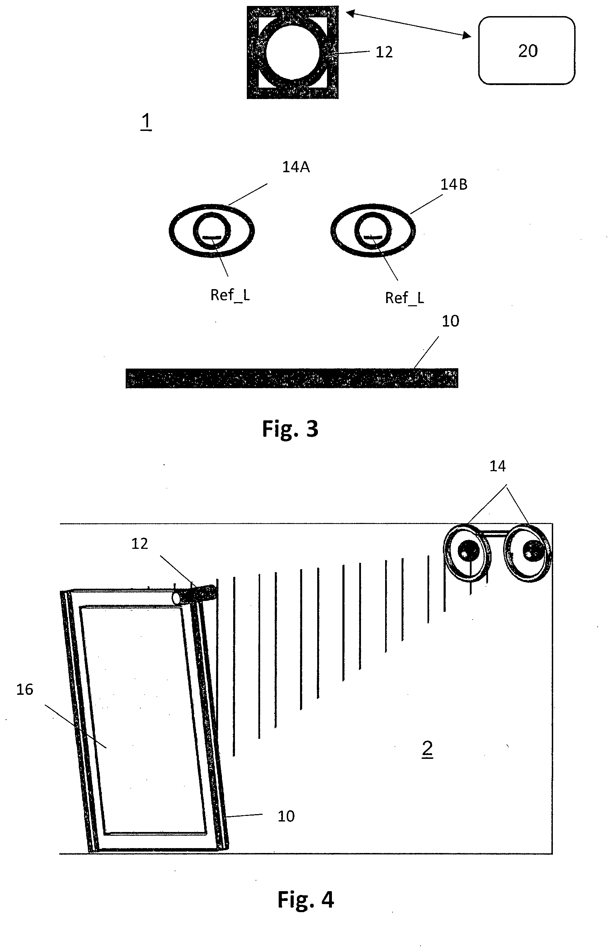

**Fig. 3** zeigt schematisch den Aufbau einer beispielhaften Vorrichtung 1 mit einer Kamera 12 (als Bildaufnahmeeinrichtung) und einer Messeinrichtung zum Bestimmen des Abstands zumindest eines der Augen eines Benutzers von der Kamera (Abstand Auge-Kamera). Die Vorrichtung 1 kann z.B. eine Vorrichtung zum Bestimmen der Parameter der Augen eines Benutzers und/oder der Gebrauchsstellung einer Brille oder einer Brillenfassung vor den Augen eines Benutzers, eine Videovermessungs- und Zentriervorrichtung (wie z.B. die Vorrichtung ImpressionIST® der Firma Rodenstock GmbH, Deutschland), eine Vorrichtung zum Anzeigen von Daten (z.B. Bilddaten) in Abhängigkeit von der Position eines Betrachters, etc. sein.

Die Vorrichtung 1 umfasst eine Beleuchtungseinrichtung mit einer Linienlichtquelle 10, die ausgelegt und eingerichtet ist, eine lineare bzw. im Wesentlichen linienförmige Lichtreflexion auf der Hornhaut der Augen 14A und 14B eines Benutzers zu erzeugen. Die Vorrichtung 1 umfasst ferner eine Bildaufnahmeeinrichtung mit einer Kamera 12, die auf die Augen 14A und 14B des Benutzers gerichtet ist. Die Linienlichtquelle 10 kann z.B. eine Leuchtstoffröhre oder eine LED Linienleuchte sein, die in einer festen und vorbekannten Position zur Kamera 12 angeordnet ist. Die Kamera 12 nimmt die Reflexionen bzw. die Spiegelungen Ref_L der Lichtquelle auf der Hornhaut der beiden Augen auf. Im Bild erscheint die Reflexion Ref_L gekrümmt, wobei die Krümmung bei konstantem Abstand des Benutzers zur Kamera abhängig vom Hornhautradius ist. Ebenso ist die Krümmung der Reflexion Ref_L im Bild abhängig vom Abstand Auge-Kamera, wobei diese Abhängigkeit deutlich geringer ist als die Abhängigkeit vom Hornhautradius. Der Abstand zwischen den Endpunkten der Reflexion Ref_L im Bild (d.h. die Länge der Reflexion Ref_L im Bild) ist abhängig vom Abstand Auge-Kamera. Ebenfalls ist die Länge der Reflexion Ref_L in erster Linie abhängig vom Abstand Auge-Kamera und in zweiter Linie abhängig vom Hornhautradius.

Die Vorrichtung 1 umfasst ferner eine Abstandsbestimmungseinrichtung 20, die ausgelegt ist, anhand des zumindest einen aufgenommenen Bildes der spekularen Reflexion der Lichtquelle den Abstand des jeweiligen Auges zur Kamera 12 zu ermitteln. Die Abstandsbestimmungseinrichtung 20 kann mittels eines geeignet programmierten Computers oder Prozessors realisiert werden. Die spekulare Reflexion der Lichtquelle auf der Hornhaut wird als spekulare Lichtreflexion bezeichnet.

**Fig. 4** zeigt schematisch eine andere beispielhafte Vorrichtung 2 mit einer Kamera und einer Messeinrichtung zum Bestimmen des Abstands Auge-Kamera, welche zur mobilen Anwendung geeignet ist. Wie bei der in Fig. 3 gezeigten Vorrichtung 1 kann die in Fig. 4 gezeigte Vorrichtung 2 eine Vorrichtung zum Bestimmen der Parameter der Augen eines Benutzers und/oder der Gebrauchsstellung einer Brille oder einer Brillenfassung vor den Augen eines Benutzers, eine Videovermessungs- und zentriervorrichtung, eine Vorrichtung zum Anzeigen von Daten (z.B. Bilddaten) in Abhängigkeit von der Position eines Betrachters, etc. sein.

Die Vorrichtung 2 kann in einem mobilen Computer, wie z.B. einem Laptop, Notebook, Tablet-Computer oder Smartphone integriert sein. Die Vorrichtung 2 umfasst eine Kamera 12, die z.B. die im mobilen Computer integrierte Kamera sein kann. Die Vorrichtung 2 umfasst ferner eine Linienlichtquelle 10, die als Beleuchtungseinrichtung für das Verfahren zum Bestimmen des Abstands zumindest eines Auges eines Benutzers von der Kamera dient. Die Linienlichtquelle 10 ist in einem festen und vorbestimmten Abstand zur Kamera 12 angeordnet. Die Linienlichtquelle 10 kann z.B. an die der Kamera 12 gegenüberliegende Seite des Gehäuses des mobilen Computers 16 angebracht werden. Sowohl die Lichtquelle 10 als auch die Kamera 12 sind auf die Augen 14 des Benutzers gerichtet. Die spekulare Reflexion der Lichtquelle 10 auf der Hornhaut der Augen 14 des Benutzers wird von der Kamera 12 aufgenommen und, wie oben beschrieben, mittels einer Abstandsbestimmungseinrichtung (nicht gezeigt) analysiert, um den Abstand Auge-Kamera zu bestimmen.

Es ist möglich, den Bildschirm 18 durch Anzeigen eines geeigneten Bildschirminhalts als Beleuchtungseinrichtung zu verwenden. Die Lichtquelle ist in diesem Fall der diffus strahlende Bildschirminhalt, zum Beispiel in Form einer hellen Linie, eines Zeichens mit linearem Abschnitt, etc., auf schwarzem oder auf homogen gefärbtem Hintergrund. In diesem Fall ist das Anbringen einer zusätzlichen Lichtquelle nicht erforderlich.

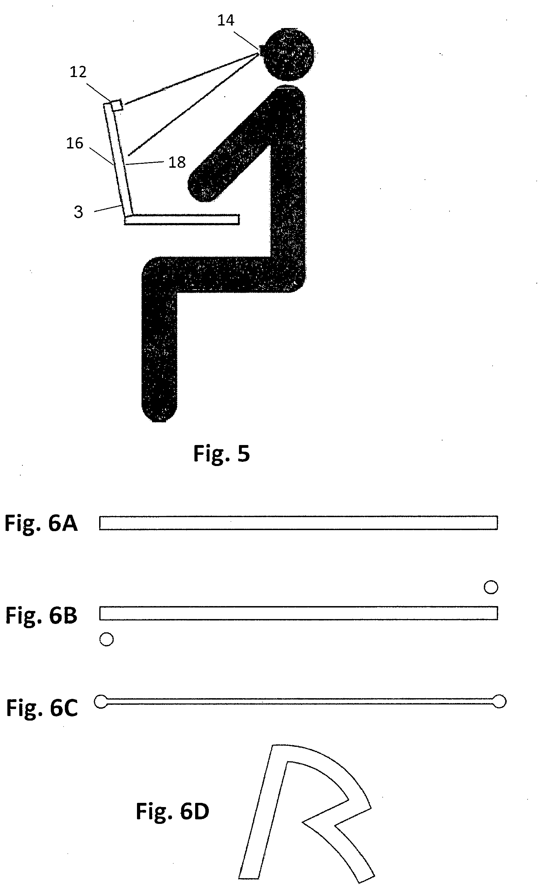

Fig. 5 zeigt eine beispielhafte Vorrichtung 3, welche in einen mobilen Computer 16 (z.B. in einen Notebook oder einen Laptop PC) integriert ist und/oder durch den mobilen Computer 16 bereitgestellt wird und welche verwendet werden kann, den Abstand des Auges bzw. der Augen 14 eines Benutzers von einer in den mobilen Computer 16 integrierten Kamera 12 zu bestimmen. In diesem Fall dient der Bildschirm 18 des mobilen Computers 16 als Beleuchtungseinrichtung. Eine auf dem Bildschirm 18 gezeigte Lichtquelle strahlt auf die Hornhaut zumindest eines der Augen 14 des Benutzers. Die spekulare Lichtreflexion auf der Hornhaut des Auges wird mittels der in dem mobilen Computer 16 integrierten Kamera 12 aufgenommen und mittels einer Abstandsbestimmungseinrichtung (nicht gezeigt) analysiert.

Die Lichtquelle kann unterschiedliche Geometrien aufweisen und somit als strukturierte Lichtquelle ausgebildet sein. Fig. 6 zeigt vier beispielhafte Lichtquellen. Fig.6A zeigt eine lineare Lichtquelle in Form eines Lichtbalkens mit vorbestimmter Länge. Fig. 6B zeigt eine Lichtquelle umfassend einen linearen Abschnitt in Form eines Lichtbalkens und zwei punktförmige bzw. kreisförmige Abschnitte an den beiden Enden des Lichtbalkens (oberhalb und unterhalb des linearen Abschnitts). Die Beleuchtungseinrichtung umfasst dementsprechend eine lineare Lichtquelle bzw. Linienlichtquelle und zwei punktförmige Lichtquellen in einer vorbestimmten Anordnung zueinander, die zusammen eine strukturierte Lichtquelle bilden. Da eine Lokalisierung der Mitten bzw. der Zentren der zwei punktförmigen bzw. kreisförmigen Abschnitte in dem aufgenommenen Bild einfacher und präziser möglich ist, wird eine genauere Abstandsmessung als mit der in Fig. 6A gezeigten Lichtquelle ermöglicht.

Fig. 6C zeigt eine Lichtquelle umfassend einen linearen Abschnitt mit punkt- bzw. kreisförmig ausgeführten Enden. Durch die Verwendung dieser Lichtquelle wird ebenfalls eine verbesserte Abstandsmessung ermöglicht. Der Vorteil dieser Anordnung gegenüber der in Fig. 6B gezeigten Anordnung ist, dass lediglich eine einzelne Lichtquelle benötigt wird. Fig. 6D zeigt eine Lichtquelle in Symbolform, hier das Unternehmenslogo der Firma Rodenstock GmbH. Die Lichtquelle weist einen linearen bzw. geraden Abschnitt auf, mittels welchem das beschriebene Verfahren durchgeführt werden kann.

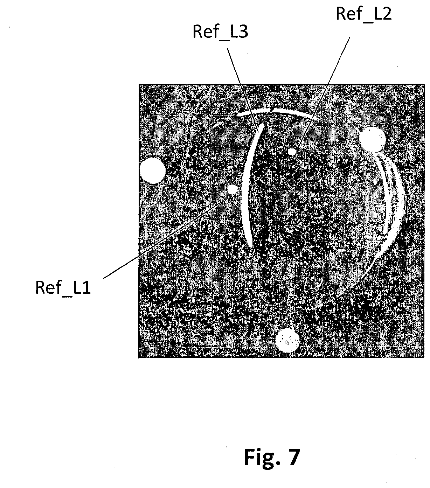

Fig. 7 zeigt eine Fotografie eines gekrümmten Glases mit einer beispielhaften Vorrichtung, die ähnlich wie die Vorrichtungen 1, 2 und/oder 3 aufgebaut und angeordnet ist. So weist die Vorrichtung eine Kamera und eine Messeinrichtung umfassend eine Linienlichtquelle L3 und zwei Punktlichtquellen L1 und L2 auf, wobei die Lichtquellen L1, L2 und L3 in einer festen und vorbekannten Position (z.B. in einem festen Abstand) relativ zur Kamera angeordnet sind. Zusammen bilden sie eine strukturierte Lichtquelle der Beleuchtungseinrichtung. Als Testobjekt bzw. Messobjekt dient das in Fig. 7 aufgenommene gekrümmte Glas als Objekt mit einer sphärischen Krümmung mit Radius r, welches das menschliche Auge simuliert.

Eine Lichtreflexion Ref_L3 der Linienlichtquelle L3 erscheint im Bild der Kamera K gekrümmt. Die Krümmung ist bei konstantem Abstand des Objekts O zur Kamera K abhängig vom Radius r des Objekts O. Ebenso ist die Krümmung der Lichtreflexion abhängig vom Abstand des Objekts O zur Kamera K. Der Abstand der Lichtreflexionen Ref_L1 und Ref_L2 der beiden Punktlichtquellen L1 und L2 ist vom Abstand des Objekts O zur Kamera K und von der Krümmung r des Objekts O abhängig (vgl. auch Gleichung 12 und 13). Da die Abhängigkeit von der jeweils zweitgenannten Größe deutlich geringer ist, kann durch Kombination der beiden Informationen aus den Reflexionen eine eindeutige Beziehung zum Abstand des Objekts O zur Kamera K und zur Krümmung r des Objekts O hergestellt werden.

Als Testobjekte können insbesondere sphärische Silikat-Gläser mit unterschiedlichen Krümmungsradien dienen. Mittels der Kamera werden in unterschiedlichen Entfernungen Bilder der verschiedenen sphärischen Silikat-Gläser aufgenommen und mittels einer Datenverarbeitungseinrichtung (nicht gezeigt) ausgewertet.

Fig. 7 zeigt eine beispielhafte Bildaufnahme eines Glases mit einem Krümmungsradius r= 39,47 mm bei einem Abstand von 37 cm der Kamera K vom Objekt O. Auf dem aufgenommenen Bild sind die Lichtreflexionen der drei Lichtquellen deutlich zu sehen.

Anhand der Bildaufnahme kann mittels einer Abstandsbestimmungseinrichtung der Abstand Testobjekt-Kamera ermittelt werden. Das Ermitteln des Abstands Testobjekt-Kamera umfasst ein Ermitteln des Abstands der Reflexionen Ref_L1 und Ref_L2 der beiden Punktlichtquellen L1 und L2 im aufgenommenen Bild. Um den Abstand der Reflexionen Ref_L1 und Ref_L2 der beiden Punktlichtquellen L1 und L2 i m aufgenommenen Bild zu ermitteln, also z.B. den Bildabstands $\Delta y''$, können zuerst manuell oder automatisch die Zentren der Reflexionen der jeweiligen Punktlichtquelle im aufgenommenen Bild ermittelt werden. Der Bildabstand $\Delta y''$ zwischen den im Bild ermittelten Zentren der beiden Reflexionen der Punktlichtquellen (z.B. in Pixeln) ist gleich dem Abstand zwischen den Reflexionen im Bild der beiden Punktlichtquellen. Aus dem Bildabstand $\Delta y''$ kann z.B. mittels Gleichung (13) der Abstand Testobjekt-Kamera ermittelt werden.

Das Ermitteln des Abstands Testobjekt-Kamera umfasst ferner ein Ermitteln der Krümmung der Lichtreflexion der Linienlichtquelle L1 im aufgenommenen Bild. Für die Krümmung der Lichtreflexion der Linienlichtquelle im Bild können Intensitäts- bzw. Helligkeitsprofile (Linienprofile) in horizontaler (oder vertikaler) Richtung über den Bereich der Reflexion im Bild erstellt werden (z.B. mit einer Breite von 1 Pixel (px)). Anders ausgedrückt können Intensitäts- bzw. Helligkeitsprofile in unterschiedlichen Schnitten (z.B. unterschiedlichen horizontalen oder vertikalen Schnitten) durch die Lichtreflexion der Lichtquelle im Bild ermittelt werden.

An jedes der ermittelten Intensitäts- bzw. Helligkeitsprofile kann jeweils eine Gaussfunktion oder eine andere geeignete Interpolationsfunktion angepasst werden. Das Maximum der angepassten Gaussfunktion gibt das Zentrum der Lichtreflexion der Linienlichtquelle L1 am Ort des jeweiligen Linienprofils an. Durch die ermittelten Zentren der Linienprofile kann eine quadratische Funktion angepasst werden, deren Faktor für die quadratische Abhängigkeit die Krümmung der Linie angibt.

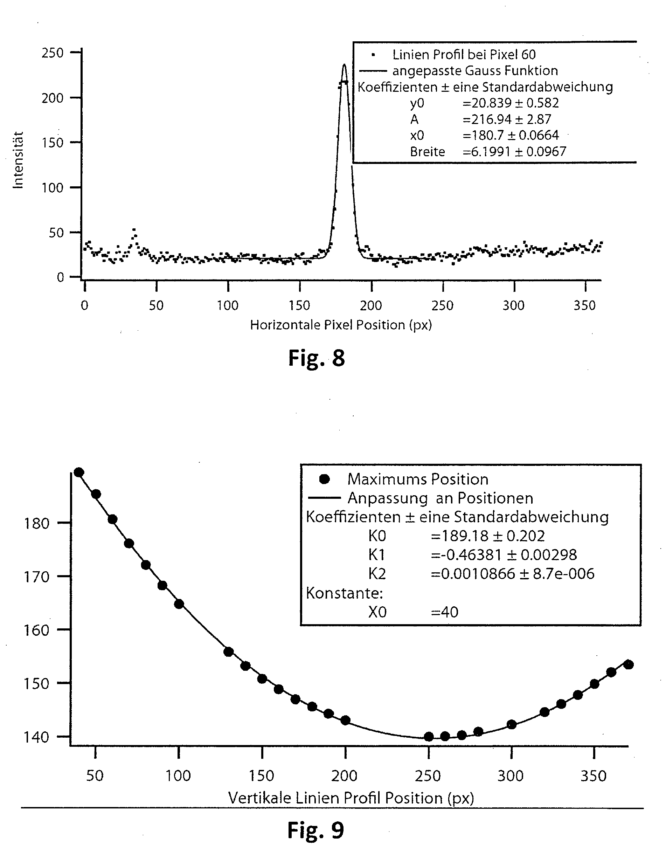

Fig. 8 zeigt ein beispielhaftes Linienprofil im aufgenommenen Bild der Hornhautreflexion der Linienlichtquelle und die angepasste Gauss-Funktion. Als Abszisse ist die horizontale Position eines Bildpunktes in Pixeln (px) und als Ordinate die Intensität bzw. Helligkeit des Bildpunkts (in Graustufenwerten) angegeben. Dabei kennzeichnet x0 den höchsten Punkt (also das Maximum) der angepassten Gaussfunktion, der als Zentrum der Lichtreflexion weiterverwendet wird. In Fig. 8 bezeichnet y0 einen Intensitätsoffset, A die Amplitude, und "width" die Breite der Gaussverteilung, welche für die Berechnung nicht weiterverwendet werden müssen.

Fig. 9 zeigt einzelne Messwerte sowie eine gefittete Funktion durch die Positionen der im Bild ermittelten Zentren der Linienprofile. Durch die gefittete Funktion wird eine angepasste quadratische Funktion bereitgestellt, bei der der Vorfaktor zum quadratischen Term und somit die Krümmung als K2 bezeichnet ist. K1 stellt den linearen Koeffizient und K0 die Konstante der angepassten quadratischen Funktion dar. Damit wird die Krümmung der Reflexion Ref_R3 durch eine Funktion ausgedrückt.

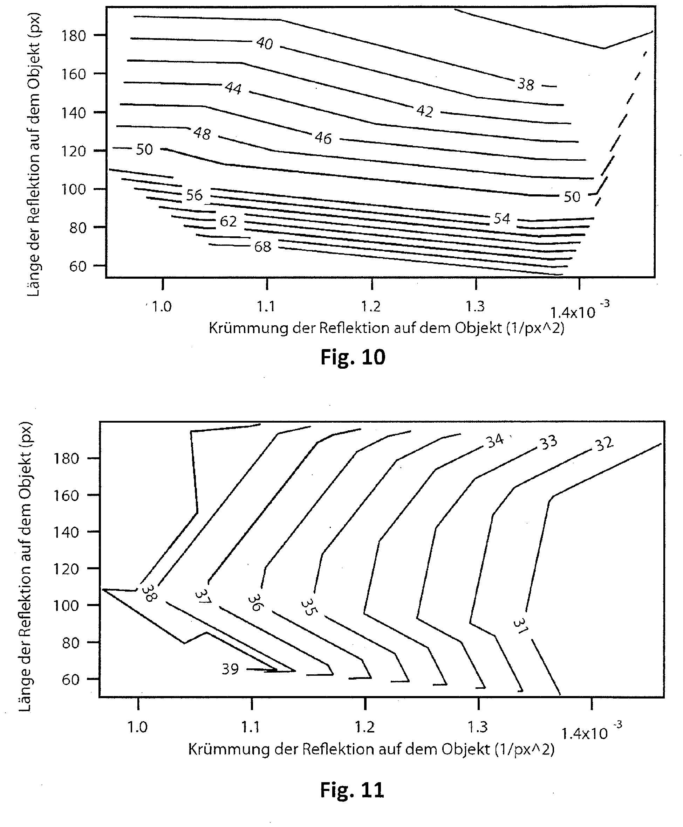

Fig. 10 und 11 zeigen jeweils die Isolinien der Objekt-Kamera Abstände in cm (Fig. 10) und die Objektkrümmungen in mm (Fig. 11). Die gemessene Krümmung der Lichtreflexion der Linienlichtquelle im aufgenommenen Bild (angegeben in $1/px^2$) ist auf die Abszisse der Fig. 10 und 11 aufgetragen. Der gemessene Abstand der Lichtreflexionen der beiden Punktlichtquellen im aufgenommenen Kamerabild in Pixeln (px) ist als Ordinate der Fig. 10 und 11 aufgetragen. Aus Fig. 10 und 11 ist ersichtlich, dass die Objektkrümmung nichtlinear vom Abstand Objekt-Kamera abhängt. Anhand der Länge und Krümmung der Lichtreflexionen der Lichtquellen im Bild können somit beide Größen (Abstand Objekt-Kamera und Objektkrümmung) eindeutig ermittelt werden.

Zur Bestimmung des Abstandes Auge-Kamera ist eine Linienlichtquelle ausreichend. Die Bestimmung kann anhand der äußersten Punkte der Linienlichtquelle erfolgen. Damit kann ein einfacher Aufbau realisiert werden.

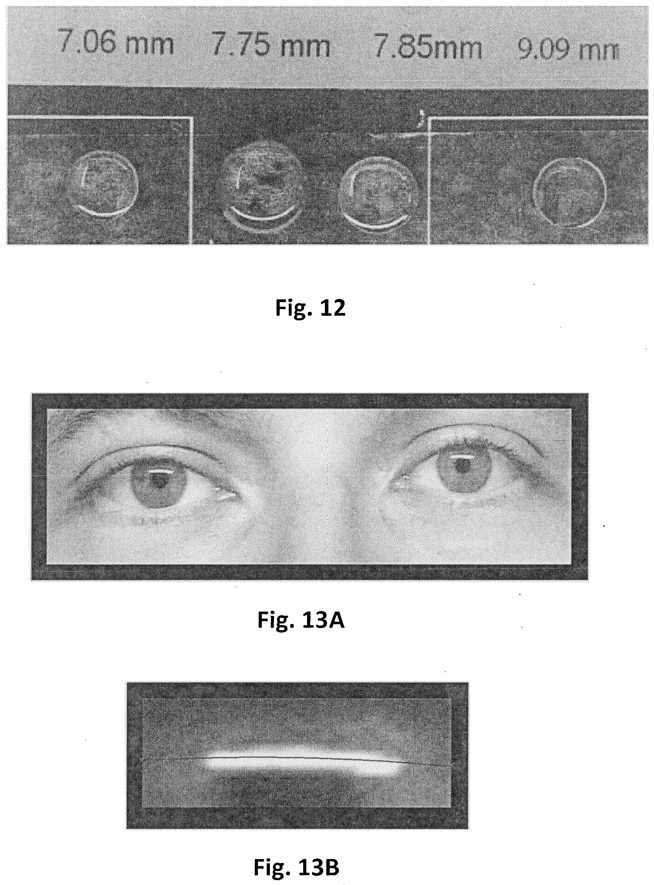

Fig. 12 zeigt Fotografien (Bildaufnahmen) von vier unbeschichteten Linsen mit unterschiedlichen Krümmungsradien zwischen 7 mm und 10 mm, nämlich 7,06 mm; 7,75 mm; 7,85 mm und 9,09 mm. Die Vorrichtung, mit der die in Fig. 12 gezeigten Bildaufnahmen erhalten worden sind, entspricht im Wesentlichen der in Fig. 4 gezeigten Vorrichtung 2.

Insbesondere weist die Vorrichtung eine im Wesentlichen gerade Linienlichtquelle auf, welche eine gekrümmte linienförmige Lichtreflexion auf der untersuchten Linse erzeugt. Die Linienlichtquelle ist an einer der langen Seiten eines Tablet-Computers befestigt ist. In diesem Beispiel ist die Länge der Linienlichtquelle 55 cm und der Abstand zwischen der im Tablet-Computer integrierten Kamera und der Linienlichtquelle ist 18 cm. Die Krümmungsradien der Linsen sind ähnlich dem Krümmungsradius der menschlichen Hornhaut (ca. 7,8 mm) und eignen sich somit als Testobjekte für die Erstellung von Kalibrierungsdaten für die Vorrichtung bzw. die in die Vorrichtung integrierte Messeinrichtung.

Die Linsen mit den Lichtreflexionen wurden bei unterschiedlichen Abständen im Bereich von 20 cm bis 75 cm zur Kamera aufgenommen. Aus den aufgenommenen Bildern wurde, wie oben beschrieben, die Krümmung und der Abstand der äußersten Punkte (also der Endpunkte) der Lichtreflexion der Linienlichtquelle ermittelt bzw. bestimmt.

Eine alleinige Bestimmung der Krümmung oder der Länge der Reflexion kann ggf. nur eine ungenaue Bestimmung des Abstands des Objekts zur Kamera erlauben. Werden sowohl Krümmung als auch Länge der Reflexion berücksichtigt, kann der Abstand des Objekts (z.B. eines Auges) zur Kamera genauer bestimmt werden.

Eine Längenbestimmung der Reflexionen der Lichtquelle in Pixeln kann dabei einen Fehler von maximal 5% der Gesamtlänge der Reflexion der Lichtquelle aufweisen, bevorzugt von maximal 2%, besonders bevorzugt von maximal 1%.

Anhand der Daten bezüglich der Länge und der Krümmung der Lichtreflexion in dem von der Kamera aufgenommenen Bild für unterschiedliche Abstände Objekt-Kamera und für Objekte mit unterschiedlichen Krümmungen können Kalibrierungsdaten ermittelt werden, mittels der die Objekt-Kamera Entfernung und die Krümmung eines unbekannten Messobjekts (wie z.B. das Auge eines Benutzers) bestimmt werden können. Die Kalibrierungsdaten können in geeigneter Form (z.B. tabellarisch, funktionell, etc.) gespeichert werden.

Die oben beschriebene Kalibrierung mittels Kalibrierungsdaten, die anhand Aufnahmen entsprechender Testobjekte verschiedener Radien in verschieden Abständen zur Kamera gewonnen werden, kann bei allen beschriebenen Vorrichtungen und Verfahren vorgenommen werden. Dazu können mehr oder weniger als vier Testobjekte verwendet werden. Die Radien der Testobjekte und deren Abstände zur Kamera können so gewählt werden, dass diese im Wesentlichen den später zu messenden Bereich abdecken. Es sind jedoch später auch Messungen möglich, die über diesen Bereich hinausgehen.

Anhand der Daten bezüglich der Länge und der Krümmung der Lichtreflexion in dem von der Kamera aufgenommenen Bild für unterschiedliche Abstände Objekt-Kamera und für Objekte mit unterschiedlichen Krümmungen kann ein Skalierungsfaktor ermittelt werden, mit welchem die im Bild gemessenen Abstände in (reale) Abstände Auge-Kamera bzw. Objekt-Kamera umgerechnet werden können. Mit der Kalibrierung bzw. mit diesem Skalierungsfaktor kann z.B. eine Messung der Pupillendistanz PD durchgeführt werden.

Die Messung der Pupillendistanz PD umfasst die Aufnahme zumindest eines Bildes der Augenpartie eines Benutzers mit der kalibrierten Kamera des Messsystems. **Fig. 13A** zeigt in einer Fotografie ein aufgenommenes Bild der Augenpartie eines Benutzers.

Die spekulare Lichtreflexion de r Linienlichtquelle kann manuell oder automatisch mittels geeigneter Bildverarbeitungsalgorithmen im aufgenommenen Bild der Augenpartie identifiziert werden. Der Bildbereich, welcher die spekulare Lichtreflexion der jeweiligen Lichtquelle enthält, kann aus dem Bild der Augenpartie ausgeschnitten und separat analysiert werden. **Fig. 13B** zeigt das ausgeschnittene Bild der spekularen Lichtreflexion der Linienlichtquelle.

Anhand der Bilddaten der spekularen Lichtreflexion können die Krümmung und die Länge der Reflexion der Linienlichtquelle im Bild manuell oder automatisch mittels geeigneter Bildverarbeitungsverfahren ermittelt werden. Die Ermittlung der Krümmung der spekularen Lichtreflexion kann die Anpassung einer quadratischen Funktion an die aufgenommene Reflexion umfassen. Die quadratische Funktion kann wie oben beschrieben, anhand von Linienprofilen in einer Mehrzahl von horizontalen oder vertikalen Schnitten durch die Lichtreflexion ermittelt werden.

Bei dem in den Figuren 13A und 13B gezeigten Beispiel kann eine Parabel mit einem quadratischen Faktor angepasst werden. Der quadratische Vorfaktor kann als Krümmungsradius verwendet werden. Die Länge der Reflexion kann subpixelgenau aus dem Bild ermittelt werden.

Durch ein Ermitteln der Werte für den Objekt-Radius und den Objekt-Kamera Abstand aus der Kalibrierung (z.B. analog zu den in den Figuren 10 und 11 gezeigten Diagrammen) kann die Position des Auges im Raum zur Kamera ermittelt werden. Für die in den Figuren 13A und 13B gezeigte Aufnahme ergeben sich somit ein bestimmbarer Abstand für das linke Auge und ein bestimmbarer Abstand für das rechte Auge.

Aus der Kalibrierung ergibt sich für diese Entfernung Auge-Kamera ein Umrechnungs- bzw. Skalierungsfaktor in Pixeln pro Millimeter [px mm$^{-1}$].

Die Pupillenmitte des jeweiligen Auges kann manuell oder automatisch mittels geeigneter Bildverarbeitungsverfahren ermittelt werden. Aus der Bildaufnahme der Augenpartie (Fig. 13A) ergibt sich ein Abstand der beiden Pupillenmitten von z.B. 350 px, abhängig von der Auflösung der Aufnahme. Unter Berücksichtigung des zuvor ermittelten Skalierungsfaktors in px/mm ergibt sich somit eine Pupillendistanz PD in mm. Bei einem bekannten Fixierpunkt relativ zur Kamera kann die Pupillendistanz mit Hilfe eines Augenmodells für den Blick nach Unendlich umgerechnet werden. So kann eine Konvergenzkorrektur für den mittleren Augenabstand (als Mittelwert des linken und rechten Augenabstands) ermittelt werden, so dass sich eine korrigierte Pupillendistanz ergibt.

Eine Verbesserung der Messgenauigkeit kann durch eine höhere Auflösung des Bildes erreicht werden. Dies kann durch einen kleineren Messabstand oder durch Verwenden eines Objektivs mit kleinerem Öffnungswinkel erzielt werden. Soll die Messvorrichtung in einen mobilen Computer integriert werden, kann für die Kamera eine Vorsatzoptik verwendet werden. Eine Verbesserung der Messgenauigkeit kann auch durch eine andere Form der Lichtquelle erreicht werden.

## Bezugszeichenliste

| | |
|---|---|
| A | Verbindungsachse |
| O | Objekt |
| K | Kamera |
| L1, L2 | Punktlichtquellen |
| L3 | Linienlichtquelle |

| Ref_L | Lichtreflexion auf der Hornhaut |
|---|---|
| Ref_L1 | Reflexion einer Punktlichtquelle auf einem Testobjekt |
| Ref_L2 | Reflexion einer Punktlichtquelle auf einem Testobjekt |
| Ref_L3 | Reflexion einer Linienlichtquelle auf einem Testobjekt |
| y1 | erster Abstand |
| y2 | zweiter Abstand |
| y1' | erster virtueller Abstand |
| y2' | zweiter virtueller Abstand |
| y1" | erster Bildebenenabstand |
| y2" | zweiter Bildebenenabstand |
| Δy | realer Abstand |
| Δy' | virtueller Bildabstand |
| Δy" | Bildabstand |
| 1, 2, 3 | Vorrichtung |
| 10 | Beleuchtungseinrichtung |
| 12 | Bildaufnahmeeinrichtung (z.B. eine Kamera) |
| 14 | Augenpaar |
| 14A, 14B | Augen |
| 16 | mobiler Computer |
| 18 | Bildschirm |
| 20 | Abstandsbestimmungseinrichtung |

**Patentansprüche**

1. Vorrichtung mit einer Bildaufnahmeeinrichtung (12; K) und einer Messeinrichtung zum Bestimmen des Abstands zumindest eines Auges (14; 14A, 14B) eines Benutzers von der Bildaufnahmeeinrichtung, wobei die Messeinrichtung umfasst:

eine Beleuchtungseinrichtung (10; L1-L3), welche in einer vorgegebenen relativen Position zur Bildaufnahmeeinrichtung (12; K) angeordnet ist, und welche ausgelegt ist, zumindest eine spekulare Lichtreflexion (Ref_L), welche einen linienförmigen Abschnitt aufweist, auf der Hornhaut des Auges (14; 14A, 14B) des Benutzers zu erzeugen; und
eine Abstandsbestimmungseinrichtung, wobei
die Bildaufnahmeeinrichtung (12; K) ausgelegt ist, zumindest ein Bild der spekularen Lichtreflexion (Ref_L) im Bereich der Hornhaut des Auges (14; 14A, 14B) des Benutzers aufzunehmen; und
die Abstandsbestimmungseinrichtung ausgelegt ist,

- Daten bezüglich des Abstands zwischen zwei vorbestimmten Punkten der Lichtreflexion (Ref_L) und der Krümmung des zumindest einen linienförmigen Abschnitts der Lichtreflexion (Ref_L) im aufgenommenen Bild zu ermitteln; und
- den Abstand des Auges (14; 14A, 14B) zur Bildaufnahmeeinrichtung (12; K) anhand der ermittelten Daten bezüglich des Abstands zwischen den zwei vorbestimmten Punkten der Lichtreflexion (Ref_L) und der Krümmung des zumindest einen linienförmigen Abschnitts der Lichtreflexion (Ref_L) zu ermitteln.

2. Vorrichtung nach Anspruch 1, wobei die Abstandsbestimmungseinrichtung dazu ausgebildet und vorgesehen ist, die zwei vorbestimmten Punkte der Lichtreflexion und den zumindest einen linienförmigen Abschnitt der Lichtreflexion automatisch im aufgenommenen Bild zu lokalisieren.

3. Vorrichtung nach Anspruch 1 oder 2, ferner umfassend:

eine Kalibrierungsdatenspeichereinrichtung, welche ausgelegt ist, Kalibrierungsdaten zu speichern, wobei die Kalibrierungsdaten Daten bezüglich einer Zuordnung von Abständen zwischen zwei vorbestimmten Punkten der Lichtreflexion (Ref_L) und von Krümmungen des zumindest einen linienförmigen Abschnitts der Lichtreflexion (Ref_L) im aufgenommenen Bild zu bestimmten Abständen von Messobjekten (O) zur Bildaufnahmeeinrichtung (12; K) enthalten; und wobei
die Abstandsbestimmungseinrichtung ausgelegt ist, den Abstand des Auges (14; 14A, 14B) zur Bildaufnahmeeinrichtung (12; K) unter Berücksichtigung der Kalibrierungsdaten zu ermitteln.

**4.** Vorrichtung nach einem der vorangegangenen Ansprüche, wobei
die Lichtreflexion (Ref_L) die Form eines Lichtbalkens aufweist; und/oder
die Lichtreflexion (Ref_L) zumindest zwei punkt- bzw. kreisförmige Bereiche aufweist; und/oder
die Lichtreflexion (Ref_L) mehrere linienförmige Abschnitte aufweist.

**5.** Vorrichtung nach einem der vorangegangenen Ansprüche, ferner umfassend eine Skalierungsfaktorbestimmungseinrichtung, welche ausgelegt ist, anhand des ermittelten Abstandes des zumindest einem Auges (14; 14A, 14B) zur Bildaufnahmeeinrichtung (12; K) zu ermitteln:

einen Skalierungsfaktor zum Umrechnen einer in einem von der Bildaufnahmeeinrichtung (12; K) aufgenommenen Bild gemessenen Distanz in eine tatsächliche Distanz; und/oder
einen Skalierungsfaktor für die Fassungsebene einer vom Benutzer getragenen Brillenfassung oder Brille.

**6.** Vorrichtung nach einem der vorangegangenen Ansprüche,
wobei die Bildaufnahmeeinrichtung (12; K) ausgelegt ist, Bilder der spekularen Lichtreflexion (Ref_L) im Bereich der Hornhaut des Auges (14; 14A, 14B) des Benutzers aus zumindest zwei unterschiedlichen Aufnahmepositionen aufzunehmen; und wobei
die Abstandsbestimmungseinrichtung ausgelegt ist, für jede der Aufnahmepositionen:

- Daten bezüglich des Abstands zwischen zwei vorbestimmten Punkten der Lichtreflexion (Ref_L) und der Krümmung des zumindest einen linienförmigen Abschnitts der Lichtreflexion (Ref_L) in dem zumindest einen aus dieser Aufnahmeposition aufgenommenen Bild zu ermitteln; und
- anhand der ermittelten Daten bezüglich des Abstands zwischen den zwei vorbestimmten Punkten der Lichtreflexion (Ref_L) und der Krümmung des zumindest einen linienförmigen Abschnitts der Lichtreflexion (Ref_L) den Abstand des Auges (14; 14A, 14B) zur Bildaufnahmeeinrichtung (12; K) für diese Aufnahmeposition zu ermitteln.

**7.** Vorrichtung nach Anspruch 6 ferner umfassend:
eine 3D-Rekonstruktionseinrichtung, welche ausgelegt ist, anhand der aus unterschiedlichen Aufnahmepositionen aufgenommenen Bilder und anhand der ermittelten Abstände des zumindest einen Auges (14; 14A, 14B) des Benutzers zur Bildaufnahmeeinrichtung (12; K) für jede der Aufnahmepositionen Ortsinformationen im dreidimensionalen Raum für zumindest einen Punkt und/oder Bereich des zumindest einen Auges des Benutzers und/oder einer vor dem Auge (14; 14A, 14B) angeordneten Brille oder Brillenfassung zu ermitteln.

**8.** Vorrichtung nach einem der vorangegangenen Ansprüche, ferner umfassend:

einen Bildschirm (18), welcher in einer vorgegebenen relativen Position zur Bildaufnahmeeinrichtung (12; K) angeordnet ist, und eine Bildschirminhaltermittlungseinrichtung, welche ausgelegt ist,

- den Abstand zwischen dem Bildschirm (18) und dem Benutzer anhand des ermittelten Abstands zwischen des zumindest einen Auges (14; 14A, 14B) des Benutzers und der Bildaufnahmeeinrichtung (12; K) zu ermitteln; und
- den auf dem Bildschirm (18) anzuzeigenden Bildschirminhalt in Abhängigkeit von dem ermittelten Abstand zwischen dem Benutzer und dem Bildschirm (18) zu verändern;

und/oder
eine Parameterbestimmungseinrichtung, welche ausgelegt ist, zumindest einen optischen Parameter des zumindest einen Auges (14; 14A, 14B) des Benutzers und/oder zumindest einen Parameter der Gebrauchsstellung einer Brille oder Brillenfassung vor den Augen (14; 14A, 14B) des Benutzers unter Berücksichtigung des ermittelten Abstandes des zumindest einen Auges (14; 14A, 14B) zur Bildaufnahmeeinrichtung (12; K) zu ermitteln.

**9.** Verfahren, umfassend:

Erzeugen einer spekularen Lichtreflexion (Ref_L) mit zumindest einem linienförmigen Abschnitt auf der Hornhaut zumindest eines Auges (14; 14A, 14B) eines Benutzers, wobei die Lichtreflexion (Ref_L) von einer Beleuchtungseinrichtung (10; L1-L3) erzeugt wird, welche in einer festen relativen Position zu einer Bildaufnahmeeinrichtung (12; K) angeordnet ist;
Aufnehmen zumindest eines Bildes der spekularen Lichtreflexion (Ref_L) im Bereich der Hornhaut des Auges

EP 3 256 036 B1

(14; 14A, 14B) des Benutzers;
Ermitteln des Abstands zwischen zwei vorbestimmten Punkten der Lichtreflexion (Ref_L) und der Krümmung des zumindest einen linienförmigen Abschnitts der Lichtreflexion (Ref_L) im aufgenommenen Bild; und
Ermitteln des Abstands des Auges (14; 14A, 14B) des Benutzers zur Bildaufnahmeeinrichtung (12; K) anhand der ermittelten Daten bezüglich des Abstands zwischen den zwei vorbestimmten Punkten der Lichtreflexion (Ref_L) und der Krümmung des zumindest einen linienförmigen Abschnitts der Lichtreflexion (Ref_L).

10. Verfahren nach Anspruch 9, ferner umfassend:

Erfassen von Kalibrierungsdaten, welche Daten bezüglich einer Zuordnung von Abständen zwischen zwei vorbestimmten Punkten der Lichtreflexion (Ref_L) und von Krümmungen des zumindest einen linienförmigen Abschnitts der Lichtreflexion (Ref_L) im aufgenommenen Bild zu bestimmten Abständen von Messobjekten zur Bildaufnahmeeinrichtung (12; K) enthalten; und
Ermitteln des Abstands des Auges (14; 14A, 14B) zur Bildaufnahmeeinrichtung (12; K) unter Berücksichtigung der Kalibrierungsdaten.

11. Verfahren nach Anspruch 9 oder 10, wobei:

die Lichtreflexion (Ref_L) die Form eines Lichtbalkens aufweist; und/oder
die Lichtreflexion (Ref_L) zumindest zwei punkt- bzw. kreisförmige Bereiche aufweist; und/oder
die Lichtreflexion (Ref_L) mehrere linienförmige Abschnitte aufweist.

12. Verfahren nach einem der Ansprüche 9 bis 11 ferner umfassend:

Ermitteln eines Skalierungsfaktors zum Umrechnen einer in einem von der Bildaufnahmeeinrichtung (12; K) aufgenommenen Bild gemessenen Distanz in eine tatsächliche Distanz und/oder
Ermitteln eines Skalierungsfaktors für die Fassungsebene einer vom Benutzer getragenen Brillenfassung oder Brille anhand des ermittelten Abstandes des zumindest einen Auges (14; 14A, 14B) zur Bildaufnahmeeinrichtung (12; K); und/oder
Ermitteln zumindest eines optischen Parameters des zumindest einen Auges (14; 14A, 14B) des Benutzers und/oder eines Parameters der Gebrauchsstellung einer Brille oder Brillenfassung vor den Augen (14; 14A, 14B) des Benutzers unter Berücksichtigung des ermittelten Abstandes des zumindest einen Auges (14; 14A, 14B) zur Bildaufnahmeeinrichtung (12; K).

13. Verfahren nach einem der Ansprüche 10 bis 12 ferner umfassend
Aufnehmen von Bildern der spekularen Lichtreflexion (Ref_L) im Bereich der Hornhaut des Auges (14; 14A, 14B) des Benutzers aus zumindest zwei unterschiedlichen Aufnahmepositionen; und
für jede der Aufnahmepositionen:

- Ermitteln des Abstands zwischen zwei vorbestimmten Punkten der Lichtreflexion (Ref_L) und der Krümmung des zumindest einen linienförmigen Abschnitts der Lichtreflexion (Ref_L) in dem aus dieser Aufnahmeposition aufgenommenen Bild; und
- Ermitteln des Abstands des Auges (14; 14A, 14B) zur Bildaufnahmeeinrichtung (12; K) bei dieser Aufnahmeposition anhand der ermittelten Daten bezüglich des Abstands zwischen den zwei vorbestimmten Punkten der Lichtreflexion (Ref_L) und der Krümmung des zumindest einen linienförmigen Abschnitts der Lichtreflexion (Ref_L).

14. Verfahren nach Anspruch 13 ferner umfassend:

Ermitteln von Ortsinformationen im dreidimensionalen Raum für zumindest einen Punkt und/oder Bereich des zumindest einen Auges (14; 14A, 14B) des Benutzers und/oder einer vor dem Auge (14; 14A, 14B) angeordneten Brille oder Brillenfassung anhand der aus unterschiedlichen Aufnahmepositionen aufgenommenen Bilder und anhand der ermittelten Abstände des zumindest einen Auges des Benutzers zur Bildaufnahmeeinrichtung (12; K) für jede Aufnahmeposition; und/oder
Ermitteln des Abstands zwischen dem Benutzer und einem Bildschirm, welcher in einer vorgegebenen relativen Position zur Bildaufnahmeeinrichtung (12; K) angeordnet ist, anhand des ermittelten Abstands zwischen dem zumindest einen Auge (14; 14A, 14B) des Benutzers und der Bildaufnahmeeinrichtung (12; K) und Verändern des auf dem Bildschirm anzuzeigenden Bildschirminhalts in Abhängigkeit von dem ermittelten Abstand des

17

zumindest einen Auges (14; 14A, 14B) des Benutzers zur Bildaufnahmeeinrichtung (12; K).

15. Computerprogrammprodukt umfassend Programmteile, welche wenn geladen in einen Computer, ausgelegt sind, ein Verfahren mit den folgenden Schritten auszuführen:

Erfassen zumindest eines Bildes einer spekularen Lichtreflexion (Ref_L) im Bereich der Hornhaut des Auges (14; 14A, 14B) eines Benutzers, wobei die Lichtreflexion (Ref_L) einen linienförmigen Abschnitt aufweist, und wobei die Lichtreflexion (Ref_L) von einer Beleuchtungseinrichtung (10; L1-L3) erzeugt wird, welche in einer festen relativen Position zu einer Bildaufnahmeeinrichtung angeordnet ist;
Ermitteln des Abstands zwischen zwei vorbestimmten Punkten der Lichtreflexion (Ref_L) und der Krümmung des zumindest einen linienförmigen Abschnitts der Lichtreflexion (Ref_L) im erfassten Bild; und
Ermitteln des Abstands des Auges (14; 14A, 14B) des Benutzers zur Bildaufnahmeeinrichtung anhand der ermittelten Daten bezüglich des Abstands zwischen den zwei vorbestimmten Punkten der Lichtreflexion (Ref_L) und der Krümmung des zumindest einen linienförmigen Abschnitts der Lichtreflexion (Ref_L).

**Claims**

1. Device with an image recording device (12; K) and a measuring device for determining the distance of at least one eye (14; 14A, 14B) of a user from the image recording device, wherein the measuring device comprises:

an illumination device (10; L 1-L3), which is arranged in a predetermined relative position to the image recording device (12; K), and which is designed to generate at least one specular light reflection (Ref_L), which has a line-shaped section, on the cornea of the user's eye (14; 14A, 14B); and
a distance determining device, wherein
the image recording device (12; K) is designed to record at least one image of the specular light reflection (Ref_L) in the area of the cornea of the user's eye (14; 14A, 14B); and
the distance determination device is designed, to

- determine data relating to the distance between two predetermined points of light reflection (Ref_L) and the curvature of the at least one line-shaped section of the light reflection (Ref_L) in the recorded image; and
- determine the distance of the eye (14; 14A, 14B) to the image recording device (12; K) based on the determined data regarding the distance between the two predetermined points of light reflection (Ref_L) and the curvature of the at least one line-shaped section of the light reflection (Ref_L).

2. Device according to claim 1, wherein the distance determination device is designed and provided to automatically locate the two predetermined points of the light reflection and the at least one line-shaped section of the light reflection in the recorded image.

3. Device according to claim 1 or 2, further comprising:

a calibration data storage device which is designed to store calibration data, wherein the calibration data include data relating to an assignment of distances between two predetermined points of the light reflection (Ref_L) and of curvatures of the at least one line-shaped section of the light reflection (Ref_L) in the recorded image at specific distances from measurement objects (O) to the image recording device (12; K); and wherein
the distance determining device is designed to determine the distance between the eye (14; 14A, 14B) and the image recording device (12; K) taking into account the calibration data.

4. Device according to one of the preceding claims, wherein
the light reflection (Ref_L) is in the form of a light bar; and / or
the light reflection (Ref_L) has at least two point or circular areas; and / or
the light reflection (Ref_L) has several linear sections.

5. Device according to one of the preceding claims, further comprising a scaling factor determination device, which is designed to determine on the basis of the determined distance of the at least one eye (14; 14A, 14B) from the image recording device (12; K):

a scaling factor for converting a distance measured in an image recorded by the image recording device (12;

K) into an actual distance; and / or
a scaling factor for the frame level of a spectacle frame or glasses worn by the user.

6. Device according to one of the preceding claims,
wherein the image recording device (12; K) is designed to take pictures of the specular light reflection (Ref_L) in the area of the cornea of the eye (14; 14A, 14B) of the user from at least two different recording positions; and wherein the distance determination device is designed for each of the recording positions:

- to determine data relating to the distance between two predetermined points of light reflection (Ref_L) and the curvature of the at least one line-shaped section of the light reflection (Ref_L) in the at least one image recorded from this recording position; and
- to determine the distance of the eye (14; 14A, 14B) to the image recording device (12; K) for this recording position, based on the determined data relating to the distance between the two predetermined points of light reflection (Ref_L) and the curvature of the at least one line-shaped section of the light reflection (Ref_L).

7. Device according to claim 6 further comprising:

a 3D-reconstruction device, which is designed to determine position information in three-dimensional space for at least one point and / or area of the at least one eye of the user and / or of a pair of glasses or spectacle frame arranged in front of the eye (14; 14A, 14B),
based on the images recorded from different recording positions and based on the determined distances of the at least one eye (14; 14A, 14B) of the user from the image recording device (12; K) for each of the recording positions.

8. Device according to one of the preceding claims, further comprising:

a screen (18), which is arranged in a predetermined relative position to the image recording device (12; K), and a screen holding device which is designed

- to determine the distance between the screen (18) and the user based on the determined distance between the at least one eye (14; 14A, 14B) of the user and the image recording device (12; K); and
- to change the screen content to be displayed on the screen (18) as a function of the determined distance between the user and the screen (18); and / or

a parameter determination device, which is designed to determine at least one optical parameter of the at least one.eye (14; 14A, 14B) of the user and / or at least one parameter of the position of use of glasses or spectacle frames in front of the eyes (14; 14A, 14B) of the user, considering the determined distance of the at least one eye (14; 14A, 14B) from the image recording device (12; K).

9. A method comprising:

Generating a specular light reflection (Ref_L) with at least one line-shaped section on the cornea of at least one eye (14; 14A, 14B) of a user, wherein the light reflection (Ref_L) is generated by an illumination device (10; L1-L3), which is arranged in a fixed relative position to an image recording device (12; K);
Recording at least one image of the specular light reflection (Ref_L) in the area of the cornea of the eye (14; 14A, 14B) of the user;
Determining the distance between two predetermined points of the light reflection (Ref_L) and the curvature of the at least one linear section of the light reflection (Ref_L) in the recorded image; and
Determining the distance of the user's eye (14; 14A, 14B) to the image recording device (12; K) based on the determined data with regard to the distance between the two predetermined points of light reflection (Ref_L) and the curvature of the at least one linear section of the light reflection (Ref_L).

10. The method according to claim 9, further comprising:

Acquisition of calibration data, which contain data relating to an assignment of distances between two predetermined points of light reflection (Ref_L) and curvatures of the at least one linear section of the light reflection (Ref_L) in the recorded image at specific distances from measurement objects to the image recording device (12; K); and

Determining the distance of the eye (14; 14A, 14B) from the image recording device (12; K) taking into account the calibration data.

11. The method according to claim 9 or 10, wherein:

the light reflection (Ref_L) is in the form of a light bar; and / or
the light reflection (Ref_L) has at least two point or circular areas; and / or
the light reflection (Ref_L) has several linear sections.

12. The method according to any one of claims 9 to 11 further comprising:

Determining a scaling factor for converting a distance measured in an image recorded by the image recording device (12; K) into an actual distance and / or
Determining a scaling factor for the frame level of a spectacle frame or glasses worn by the user on the basis of the determined distance of the at least one eye (14; 14A, 14B) from the image recording device (12; K); and / or
Determining at least one optical parameter of the at least one eye (14; 14A, 14B) of the user and / or a parameter of the position of use of glasses or spectacle frames in front of the eyes (14; 14A, 14B) of the user, taking into account the determined distance of the at least one eye (14; 14A, 14B) to the image recording device (12; K).

13. The method according to any one of claims 10 to 12 further comprising Recording images of the specular light reflection (Ref_L) in the area of the cornea of the eye (14; 14A, 14B) of the user from at least two different recording positions; and
for each of the shooting positions:

- Determining the distance between two predetermined points of light reflection (Ref_L) and the curvature of the at least one linear section of the light reflection (Ref_L) in the image recorded from this recording position; and
- Determining the distance of the eye (14; 14A, 14B) to the image recording device (12; K) in this recording position on the basis of the determined data relating to the distance between the two predetermined points of light reflection (Ref_L) and the curvature of the at least one linear section of the light reflection (Ref_L).

14. The method according to claim 13 further comprising:

Determining location information in three-dimensional space for at least one point and / or area of the at least one eye (14; 14A, 14B) of the user and / or of a pair of glasses or spectacle frames arranged in front of the eye (14; 14A, 14B) based on images, which were recorded at different recording positions and based on the determined distances of the at least one eye of the user to the image recording device (12; K) for each recording position; and / or
Determining the distance between the user and a screen, which is arranged in a predetermined relative position to the image recording device (12; K), based on the determined distance between the at least one eye (14; 14A, 1 4B) of the user and the image recording device (12; K); and
Changing the screen content to be displayed on the screen as a function of the determined distance of the at least one eye (14; 14A, 14B) of the user from the image recording device (12; K).

15. Computer program product comprising program parts which, when loaded into a computer, are designed to carry out a method with the following steps:

Acquiring at least one image of a specular light reflection (Ref_L) in the area of the cornea of the eye (14; 14A, 14B) of a user, wherein the light reflection (Ref_L) has a line-shaped section, and wherein the light reflection (Ref_L) from an illumination device (10; L1-L3) is generated, which is arranged in a fixed relative position to an image recording device;
Determining the distance between two predetermined points of the light reflection (Ref_L) and the curvature of the at least one linear section of the light reflection (Ref_L) in the captured image; and
Determining the distance of the user's eye (14; 14A, 14B) to the image recording device based on the determined data with regard to the distance between the two predetermined points of light reflection (Ref_L) and the curvature of the at least one linear section of the light reflection (Ref_L).

**Revendications**

1. Dispositif avec un dispositif d'enregistrement d'images (12; K) et un dispositif de mesure pour déterminer la distance d'au moins un œil (14; 14A, 14B) d'un utilisateur par rapport au dispositif d'enregistrement d'images, dans lequel le dispositif de mesure comprend:

   un dispositif d'éclairage (10; L1-L3), qui est disposé dans une position relative prédéterminée par rapport au dispositif d'enregistrement d'image (12; K), et qui est conçu pour générer au moins une réflexion de lumière spéculaire (Ref_L), qui a une section en forme de ligne, sur la cornée de l'œil de l'utilisateur (14; 14A, 14B); et
   un dispositif de détermination de distance, dans lequel
   le dispositif d'enregistrement d'image (12; K) est conçu pour enregistrer au moins une image de la réflexion de la lumière spéculaire (Ref_L) dans la zone de la cornée de l'œil de l'utilisateur (14; 14A, 14B); et
   le dispositif de détermination de distance est conçu pour

   - déterminer des données relatives à la distance entre deux points de réflexion de la lumière prédéterminés (Ref_L) et la courbure de l'au moins une section en forme de ligne de la réflexion de la lumière (Ref_L) dans l'image enregistrée; et
   - déterminer la distance de l'œil (14; 14A, 14B) au dispositif d'enregistrement d'image (12; K) sur la base des données déterminées concernant la distance entre les deux points prédéterminés de réflexion de la lumière (Ref_L) et la courbure de l'au moins une section en forme de ligne de la réflexion de la lumière (Ref_L),

2. Dispositif selon la revendication 1, dans lequel le dispositif de détermination de distance est conçu et prévu pour localiser automatiquement les deux points prédéterminés de la réflexion de la lumière et l'au moins une section en forme de ligne de la réflexion de la lumière dans l'image enregistrée.

3. Dispositif selon la revendication 1 ou 2, comprenant en outre:

   un dispositif de stockage de données d'étalonnage qui est conçu pour stocker des données d'étalonnage, dans lequel les données d'étalonnage comprennent des données relatives à une affectation de distances entre deux points prédéterminés de la réflexion de la lumière (Ref_L) et des courbures de l'au moins une section en forme de ligne de la réflexion de la lumière (Ref_L) dans l'image enregistrée à des distances spécifiques des objets de mesure (O) au dispositif d'enregistrement d'image (12; K); et dans lequel
   le dispositif de détermination de distance est conçu pour déterminer la distance entre l'œil (14; 14A, 14B) et le dispositif d'enregistrement d'image (12; K) en tenant compte des données d'étalonnage.

4. Dispositif selon l'une des revendications précédentes, dans lequel
   la réflexion lumineuse (Ref_L) se présente sous la forme d'une barre lumineuse; et / ou
   la réflexion lumineuse (Ref_L) a au moins deux zones ponctuelles ou circulaires; et / ou
   la réflexion lumineuse (Ref_L) a plusieurs sections linéaires.

5. Dispositif selon l'une des revendications précédentes, comprenant en outre un dispositif de détermination de facteur d'échelle, qui est conçu pour déterminer sur la base de la distance déterminée de l'au moins un œil (14; 14A, 14B) par rapport au dispositif d'enregistrement d'images (12; K):

   un facteur d'échelle pour convertir une distance mesurée dans une image enregistrée par le dispositif d'enregistrement d'image (12; K) en une distance réelle; et / ou
   un facteur d'échelle pour le niveau de monture d'une monture de lunettes ou des lunettes portées par l'utilisateur.

6. Dispositif selon l'une des revendications précédentes,
   dans lequel le dispositif d'enregistrement d'image (12; K) est conçu pour prendre des photos de la réflexion de la lumière spéculaire (Ref_L) dans la zone de la cornée de l'œil (14; 14A, 14B) de l'utilisateur à partir d'au moins deux positions d'enregistrement différentes; et dans lequel
   le dispositif de détermination de la distance est conçu pour chacune des positions d'enregistrement:

   - pour déterminer des données relatives à la distance entre deux points de réflexion de la lumière prédéterminés (Ref_L) et la courbure de l'au moins une section en forme de ligne de la réflexion de la lumière (Ref_L) dans l'au moins une image enregistrée à partir de cette position d'enregistrement; et

- pour déterminer la distance de l'œil (14; 14A, 14B) au dispositif d'enregistrement d'image (12; K) pour cette position d'enregistrement, sur la base des données déterminées relatives à la distance entre les deux points de réflexion de la lumière prédéterminés (Ref_L) et la courbure de l'au moins une section en forme de ligne de la réflexion de la lumière (Ref_L).

**7.** Dispositif selon la revendication 6 comprenant en outre:

un dispositif de reconstruction 3D, qui est conçu pour déterminer des informations de position dans un espace tridimensionnel pour au moins un point et / ou une zone du au moins un œil de l'utilisateur et / ou d'une paire de lunettes ou monture de lunettes disposée devant l'œil (14; 14A, 14B),
sur la base des images enregistrées à partir de différentes positions d'enregistrement et sur la base des distances déterminées de au moins un œil (14; 14A, 14B) de l'utilisateur par rapport au dispositif d'enregistrement d'image (12; K) pour chacune des positions d'enregistrement.

**8.** Dispositif selon l'une des revendications précédentes, comprenant en outre:

un écran (18), qui est disposé dans une position relative prédéterminée par rapport au dispositif d'enregistrement d'image (12; K), et un dispositif de maintien d'écran qui est conçu

- pour déterminer la distance entre l'écran (18) et l'utilisateur sur la base de la distance déterminée entre le au moins un œil (14; 14A, 14B) de l'utilisateur et le dispositif d'enregistrement d'image (12; K); et
- modifier le contenu de l'écran à afficher sur l'écran (18) en fonction de la distance déterminée entre l'utilisateur et l'écran (18); et / ou

un dispositif de détermination de paramètres, qui est conçu pour déterminer au moins un paramètre optique d'au moins un œil (14; 14A, 14B) de l'utilisateur et / ou au moins un paramètre de la position d'utilisation des lunettes ou des montures de lunettes devant les yeux (14; 14A, 14B) de l'utilisateur, compte tenu de la distance déterminée du au moins un œil (14; 14A, 14B) par rapport au dispositif d'enregistrement d'image (12; K).

**9.** Procédé comprenant:

Génération d'une réflexion de lumière spéculaire (Ref_L) avec au moins une section en forme de ligne sur la cornée d'au moins un œil (14; 14A, 14B) d'un utilisateur, dans laquelle la réflexion de la lumière (Ref_L) est générée par un dispositif d'éclairage (10 ; L1-L3), qui est disposé dans une position relative fixe par rapport à un dispositif d'enregistrement d'image (12; K);
Enregistrer au moins une image de la réflexion de la lumière spéculaire (Ref_L) dans la zone de la cornée de l'œil (14; 14A, 14B) de l'utilisateur;
Déterminer la distance entre deux points prédéterminés de la réflexion lumineuse (Ref_L) et la courbure de l'au moins une section linéaire de la réflexion lumineuse (Ref_L) dans l'image enregistrée; et
Déterminer la distance de l'œil de l'utilisateur (14; 14A, 14B) au dispositif d'enregistrement d'image (12; K) sur la base des données déterminées en ce qui concerne la distance entre les deux points de réflexion de la lumière prédéterminés (Ref_L) et la courbure du au moins une section linéaire de la réflexion lumineuse (Ref_L).

**10.** Procédé selon la revendication 9, comprenant en outre:

Acquisition de données d'étalonnage, qui contiennent des données relatives à une affectation de distances entre deux points de réflexion de la lumière prédéterminés (Ref_L) et des courbures de l'au moins une section linéaire de la réflexion de la lumière (Ref_L) dans l'image enregistrée à des distances spécifiques des objets de mesure au dispositif d'enregistrement d'image (12; K); et
Déterminer la distance de l'œil (14; 14A, 14B) du dispositif d'enregistrement d'image (12; K) en tenant compte des données d'étalonnage.

**11.** Procédé selon la revendication 9 ou 10, dans lequel:

la réflexion lumineuse (Ref_L) se présente sous la forme d'une barre lumineuse; et / ou
la réflexion lumineuse (Ref_L) a au moins deux zones ponctuelles ou circulaires; et / ou
la réflexion lumineuse (Ref_L) a plusieurs sections linéaires.

**12.** Procédé selon l'une quelconque des revendications 9 à 11, comprenant en outre:

Détermination d'un facteur d'échelle pour convertir une distance mesurée dans une image enregistrée par le dispositif d'enregistrement d'image (12; K) en une distance réelle et / ou Déterminer un facteur d'échelle pour le niveau de monture d'une monture de lunettes ou de lunettes portées par l'utilisateur sur la base de la distance déterminée de l'au moins un œil (14; 14A, 14B) du dispositif d'enregistrement d'image (12; K); et / ou
Déterminer au moins un paramètre optique de l'au moins un œil (14; 14A, 14B) de l'utilisateur et / ou un paramètre de la position d'utilisation des lunettes ou montures de lunettes devant les yeux (14; 14A, 14B) de l'utilisateur, en tenant compte de la distance déterminée du au moins un œil (14; 14A, 14B) au dispositif d'enregistrement d'image (12; K).

**13.** Procédé selon l'une quelconque des revendications 10 à 12, comprenant en outre Enregistrer des images de la réflexion de la lumière spéculaire (Ref_L) dans la zone de la cornée de l'œil (14; 14A, 14B) de l'utilisateur à partir d'au moins deux positions d'enregistrement différentes; et

pour chacune des positions de tir:

- déterminer la distance entre deux points prédéterminés de réflexion lumineuse (Ref_L) et la courbure de l'au moins une section linéaire de la réflexion lumineuse (Ref_L) dans l'image enregistrée à partir de cette position d'enregistrement; et
- détermination de la distance de l'oeil (14; 14A, 14B) au dispositif d'enregistrement d'image (12; K) dans cette position d'enregistrement sur la base des données déterminées relatives à la distance entre les deux points de réflexion de la lumière prédéterminés (Ref_L) et la courbure de l'au moins une section linéaire de la réflexion lumineuse (Ref_L).

**14.** Procédé selon la revendication 13, comprenant en outre:

Détermination d'informations de localisation dans un espace tridimensionnel pour au moins un point et / ou une zone d'au moins un œil (14; 14A, 14B) de l'utilisateur et / ou d'une paire de lunettes ou montures de lunettes disposées devant l'œil (14; 14A, 14B) sur la base d'images, qui ont été enregistrées à différentes positions d'enregistrement et sur la base des distances déterminées d'au moins un œil de l'utilisateur au dispositif d'enregistrement d'image (12; K) pour chaque position d'enregistrement; et / ou
Déterminer la distance entre l'utilisateur et un écran, qui est disposé dans une position relative prédéterminée par rapport au dispositif d'enregistrement d'image (12; K), sur la base de la distance déterminée entre l'au moins un œil (14; 14A, 1 4B) de l'utilisateur et le dispositif d'enregistrement d'image (12; K); et
Modification du contenu de l'écran à afficher sur l'écran en fonction de la distance déterminée d'au moins un œil (14; 14A, 14B) de l'utilisateur par rapport au dispositif d'enregistrement d'image (12; K).

**15.** Produit de programme informatique comprenant des parties de programme qui, une fois chargées dans un ordinateur, sont conçues pour exécuter un procédé comprenant les étapes suivantes:

Acquisition d'au moins une image d'une réflexion de la lumière spéculaire (Ref_L) dans la zone de la cornée de l'œil (14; 14A, 14B) d'un utilisateur, dans laquelle la réflexion de la lumière (Ref_L) a une section en forme de ligne, et dans laquelle la réflexion de la lumière (Ref_L) provenant d'un dispositif d'éclairage (10; L1-L3) est générée, qui est disposée dans une position relative fixe par rapport à un dispositif d'enregistrement d'image;
Déterminer la distance entre deux points prédéterminés de la réflexion de la lumière (Ref_L) et la courbure de l'au moins une section linéaire de la réflexion de la lumière (Ref_L) dans l'image capturée; et
Détermination de la distance de l'œil de l'utilisateur (14; 14A, 14B) au dispositif d'enregistrement d'image sur la base des données déterminées en ce qui concerne la distance entre les deux points de réflexion de la lumière prédéterminés (Ref_L) et la courbure de l'au moins une section linéaire de la réflexion lumineuse (Ref_L).

**Fig. 1**

**Fig. 2**

**Fig. 3**

**Fig. 4**

**Fig. 5**

**Fig. 6A**

**Fig. 6B**

**Fig. 6C**

**Fig. 6D**

**Fig. 7**

**Fig. 8**

**Fig. 9**

Fig. 10

Fig. 11

**Fig. 12**

**Fig. 13A**

**Fig. 13B**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102005003699 A1
- DE 102014009459
- DE 102005038218 A1
- WO 2008009355 A
- WO 2014103646 A
- DE 102014015671

### In der Beschreibung aufgeführte Nicht-Patentliteratur

- **DR. ROLAND ENDERS.** Die Optik des Auges und der Sehhilfen. Optische Fachveröffentlichung GmbH, 1995